(19) Europäisches Patentamt
European Patent Office
Office européen des brevets

(11) **EP 4 106 863 B1**

(12) **EUROPEAN PATENT SPECIFICATION**

(45) Date of publication and mention
of the grant of the patent:
**09.04.2025   Bulletin 2025/15**

(21) Application number: **21756725.4**

(22) Date of filing: **19.02.2021**

(51) International Patent Classification (IPC):
*A61N 1/378* (2006.01)        *A61N 1/00* (2006.01)
*A61N 1/36* (2006.01)         *A61B 5/00* (2006.01)
*A61N 1/05* (2006.01)

(52) Cooperative Patent Classification (CPC):
**A61N 1/3787; A61N 1/3605;** A61B 5/0031;
A61B 5/293; A61B 5/294; A61B 5/296;
A61B 5/4064; A61B 2560/0219; A61B 2562/0209;
A61N 1/0529

(86) International application number:
**PCT/US2021/018644**

(87) International publication number:
**WO 2021/168163 (26.08.2021 Gazette 2021/34)**

(54) **ANCHOR LOSS IN MILLIMETER-SCALE ULTRASONIC WIRELESS IMPLANTABLE DEVICES**

VERANKERUNGSVERLUST IM MILLIMETERBEREICH IN DRAHTLOSEN IMPLANTIERBAREN ULTRASCHALLVORRICHTUNGEN

PERTE D'ANCRAGE DANS DES DISPOSITIFS IMPLANTABLES SANS FIL ULTRASONORES À L'ÉCHELLE MILLIMÉTRIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.02.2020   US 202062978520 P**

(43) Date of publication of application:
**28.12.2022   Bulletin 2022/52**

(73) Proprietors:
• **Chan Zuckerberg Biohub, Inc.
San Francisco, CA 94158 (US)**
• **The Regents of the University of California
Oakland, CA 94607-5200 (US)**

(72) Inventors:
• **MAHARBIZ, Michel, M.
Oakland, CA 94607-5200 (US)**
• **SONMEZOGLU, Soner
Oakland, California, CA 94607-5200 (US)**

(74) Representative: **Mewburn Ellis LLP
Aurora Building
Counterslip
Bristol BS1 6BX (GB)**

(56) References cited:
WO-A1-2019/204773        WO-A2-2005/103873
US-A- 5 279 292          US-A1- 2008 108 915
US-A1- 2017 125 892      US-A1- 2018 085 605
US-A1- 2018 193 000

**Description**

**FIELD OF THE DISCLOSURE**

The present invention relates to ultrasonic wireless implantable devices. More particularly, the present invention relates to attaching a piezoelectric crystal to a substrate of an ultrasonic wireless implantable device in a manner that reduces energy loss due to anchor loss.

**BACKGROUND**

Acoustic links for medical implants have gained attention primarily because they provide a route to wireless deep tissue systems. The miniaturization of the implants is often another key research goal in these efforts, nominally because smaller implants result in less acute, and possible chronic, tissue damage. Implant size in acoustic systems is limited in part by the onboard piezoelectric bulk crystal used for power harvesting and data communication.

Piezoelectric crystals serve as a critical component in the construction of ultrasonic wireless implantable devices; these crystals convert mechanical (acoustic) energy into electrical energy and vice versa by the piezoelectric effect. Millimeter-scale ultrasonic wireless implantable devices consist of at least a piezoelectric crystal and an integrated circuit (IC), where the piezoelectric crystal is used to power the IC and communicate with an external transceiver. Recently, many researchers in the bioelectronics field have focused on interrogating these devices implanted in deep areas of the body. A key challenge to operate these devices at centimeter-scale depths in the body is to deliver enough ultrasound energy to an implant without exceeding FDA-regulated safe power limits. In particular, miniaturization of the piezocrystal can degrade system power transfer efficiency and data transfer reliability.

A way to overcome this challenge is to maximize acoustic-to-electrical power conversion efficiency of the piezoelectric crystal. Existing ultrasonic implantable devices suffer from low acoustic-to-electrical power conversion efficiency of the piezoelectric crystal which is mainly limited by mechanical quality factor of the piezoelectric crystal, as the power conversion efficiency of the piezoelectric crystal is directly proportional to its mechanical quality factor (Q). Generally speaking, the piezoelectric crystal needs to have very low energy loss to achieve a high mechanical quality factor.

One source of energy loss in a piezoelectric crystal is "anchor loss" - a loss in vibration energy generated by the piezoelectric crystal due to "anchoring" the crystal to the substrate to which it is attached.

WO2019/204773 discloses the preamble of independent claim 1.

**SUMMARY**

The invention is defined by the independent claim 1. The present disclosure provides a new method for packaging the implant piezocrystal that maximizes power harvesting efficiency ($\eta$) and information transfer across the acoustic link. The method relies on placing anchors to the piezo regions where the vibrational mode displacement is zero. In particular, described herein are ultrasonic wireless implantable devices having a piezoelectric crystal attached to the substrate of the ultrasonic wireless implantable device via electrodes in a manner that reduces vibration or oscillation energy loss due to anchor loss. In one aspect, an implantable device is provided, comprising: a substrate; an integrated circuit attached to the substrate; and an ultrasonic transducer configured to receive ultrasonic waves that power the integrated circuit, wherein the ultrasonic transducer is attached to the substrate via one or more electrodes, and wherein the total electrode surface area in contact with the ultrasonic transducer is smaller than the surface area of a face of the ultrasonic transducer to which the one or more electrodes are attached.

In some embodiments, the one or more electrodes are each positioned at a respective corner of the face of the ultrasonic transducer to which the one or more electrodes are attached. In some embodiments, the one or more electrodes are each positioned at an edge of the face of the ultrasonic transducer to which the one or more electrodes are attached. In some embodiments, the one or more electrodes comprise a single electrode positioned at a center of the face of the ultrasonic transducer to which the single electrode is attached.

In some embodiments, the one or more electrodes include an electrode having an opening between the face of the ultrasonic transducer and the substrate.

In some embodiments, the ultrasonic transducer has a cubic shape. In some embodiments, the ultrasonic transducer has a rectangular prism shape. In some embodiments, the face of the ultrasonic transducer to which the one or more electrodes are attached has a rectangular shape.

In some embodiments, a ratio of a length of each of the one or more electrodes to a length of the face of the ultrasonic transducer to which the one or more electrodes are attached is less than 0.1. In some embodiments, a ratio of a length of each of the one or more electrodes to a length of the face of the ultrasonic transducer to which the one or more electrodes are attached is less than 0.2.

In some embodiments, the ultrasonic transducer is further configured to emit an ultrasonic backscatter.

In some embodiments, the ultrasonic transducer is configured to receive the ultrasonic waves from an interrogator comprising one or more additional ultrasonic transducers.

In some embodiments, the ultrasonic transducer is a bulk piezoelectric transducer.

In some embodiments, the implantable device is implanted in a subject. In some embodiments, the subject is a human. In some embodiments, the subject is an animal or a plant.

In some embodiments, the implantable device is about 5 mm or less in length in the longest dimension. In some embodiments, the implantable device has a volume of about 5 mm or smaller. In some embodiments, the implanted device is at least partially encapsulated by a biocompatible material.

In another aspect, a system comprising one or more of such implantable devices is provided, as well as an interrogator comprising one or more ultrasonic transducers configured to transmit ultrasonic waves to the one or more implantable devices or receive ultrasonic backscatter from the one or more implantable devices.

In some embodiments, the interrogator comprises one or more ultrasonic transducer arrays, wherein each transducer array comprises two or more ultrasonic transducers.

In some embodiments, the system comprises a plurality of implantable devices.

In some embodiments, the interrogator is configured to beam steer transmitted ultrasonic waves to alternatively focus the transmitted ultrasonic waves on a first portion of the plurality of implantable devices or focus the transmitted ultrasonic waves on a second portion of the plurality of implantable devices.

In some embodiments, the interrogator is configured to simultaneously receive ultrasonic backscatter from at least two implantable devices.

In some embodiments, the interrogator is configured to transit ultrasonic waves to the plurality of implantable devices or receive ultrasonic backscatter from the plurality of implantable devices using time division multiplexing, spatial multiplexing, or frequency multiplexing.

In some embodiments, the interrogator is configured to be wearable by a subject.

## BRIEF DESCRIPTION OF THE DRAWINGS

FIG. 1 is a schematic of a neural dust system, including an external transceiver, a subdural interrogator, and a neural dust mote.

FIG. 2A is a block diagram of an exemplary interrogator for a system described herein. The illustrated interrogator includes an ultrasonic transducer array comprising a plurality of ultrasonic transducers. Each of the ultrasonic transducers in the array is operated by a channel, which includes a switch to alternatively configure the transducer to receive or transmit ultrasonic waves.

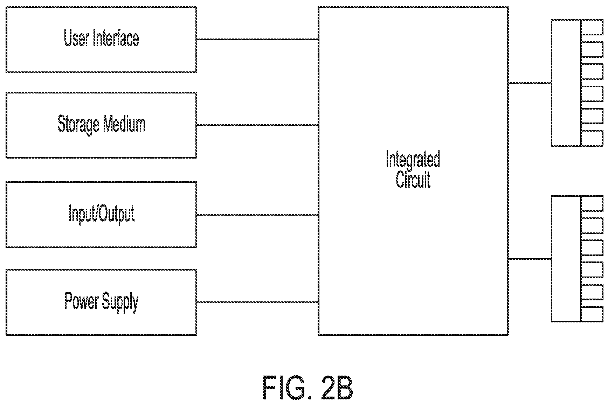

FIG. 2B is a schematic of another exemplary interrogator for a system described herein. The illustrated interrogator includes two ultrasonic transducer arrays, with each ultrasonic transducer array including a plurality of ultrasonic transducers. The interrogator also includes an integrated circuit (which can include a digital circuit, which can include a processor). The integrated circuit is connected to a user interface (which can include a display, keyboard, buttons, etc.), a storage medium (i.e., a non-transitory memory), an input/output (which may be wireless, such as a Bluetooth), and a power supply (such as a battery).

FIG. 3A shows a block diagram of an exemplary interrogator that can be worn by a subject. The interrogator includes a wireless communication system (a Bluetooth radio, in the illustration), which can be used to communicate with a computer system.

FIG. 3B shows an exploded view of a wearable interrogator. The interrogator includes a battery, a wireless communication system, and a transducer array.

FIG. 3C shows the wearable interrogator shown in FIG. 3B fully assembled with a harness for attachment to a subject.

FIG. 3D illustrates the wearable interrogator attached a subject, namely a rodent (although could be any other animal, such as a human, dog, cat, horse, cow, pig, sheep, goat, chicken, monkey, rat or mouse). The interrogator includes a transducer array, which is fixed to the body of the subject by an adhesive.

FIG. 3E illustrates a cross-section of the transducer array of the interrogator shown in FIGS. 3A-D.

FIG. 4 provides a schematic showing the communication between a transducer from an interrogator and an implantable device having a miniaturized ultrasonic transducer. The interrogator transmits ultrasonic waves to the implantable device, and the miniaturized ultrasonic transducer emits ultrasonic backscatter modulated by the sensor. The backscatter is then received by the interrogator.

FIG. 5A shows a series of cycles of ultrasonic wave pulses emitted by an interrogator. Upon receiving a trigger from the interrogator (e.g., an FPGA), the transceiver board of the interrogator generates a series of transmit pulses. At the end of the transmit cycle, the switch on the ASIC disconnects the transmit module and connects the receive module. The cycles have a frequency of every 100 microseconds.

FIG. 5B shows a zoomed-in view of the transmit pulse sequence (i.e., one cycle) shown in FIG. 5A, with the cycle having six pulses of ultrasonic waves at 1.85 MHz, the pulses recurring every 540 nanoseconds.

FIG. 5C shows ultrasonic backscatter emitted by an implantable device. The ultrasonic backscatter reaches the transducer of the interrogator approximately 2tRayleigh.

FIG. 5D shows a zoomed-in view of the ultrasonic backscatter, which can be analyzed. Analysis of the ultrasonic backscatter can include filtering, rectifying and integrating the ultrasonic backscatter waves.

FIG. 5E shows a zoomed in view of the filtered ultrasonic backscatter waves. The backscatter wave includes responsive regions, which are responsive to changes in impedance to the miniaturized ultrasonic transducer, and non- responsive regions that are not responsive to changes in impedance to the miniaturized ultrasonic transducer.

FIG. 6 illustrates one embodiment of an ASIC attached to a miniaturized ultrasonic transducer for an implantable device.

FIG. 7A illustrates a schematic of an exemplary implantable device including a miniaturized ultrasonic transducer and an ASIC on a printed circuit board (PCB).

FIG. 7B illustrates a schematic of another exemplary implantable device including a miniaturized ultrasonic transducer and an ASIC on a printed circuit board (PCB).

FIG. 8 illustrates a method of manufacturing an implantable device described herein.

FIG. 9 is a flowchart for a method of encapsulating an implantable device with amorphous silicon carbide.

FIG. 10A is a heat map illustrating example deformation in a cubic piezoelectric crystal fully "anchored" to a substrate of an ultrasonic wireless implantable device.

FIG. 10B is a heat map illustrating example deformation in a cubic piezoelectric crystal anchored to the substrate of the ultrasonic wireless implantable device by an electrode positioned at the corners of one face of the cubic piezoelectric crystal.

FIG. 11A is a heat map illustrating example deformation in a cubic piezoelectric crystal anchored to the substrate of the ultrasonic wireless implantable device by electrodes positioned at the corners of one face of the cubic piezoelectric crystal.

FIG. 11B illustrates an example of how electrodes could be positioned at the corners of one face of a cubic piezoelectric crystal to attach the face of the cubic piezoelectric crystal to a substrate.

FIG. 11C is an example graph illustrating a change in mechanical quality factor (Q) of the piezoelectric crystal as the length "L" of each corner electrode (as shown in FIG. 11B) is increased.

FIG. 11D is an example graph illustrating a normalized amplitude of energy or voltage produced by a $1 \times 1 \times 1$ mm$^3$ piezoelectric crystal attached to an ultrasonic wireless implantable device via corner electrodes (as shown in FIG. 11B) having various lengths "L" (and corresponding various mechanical factors (Q)) at a range of frequencies.

FIG. 11E is an example graph illustrating a normalized amplitude of energy or voltage produced by a $0.75 \times 0.75 \times 0.75$

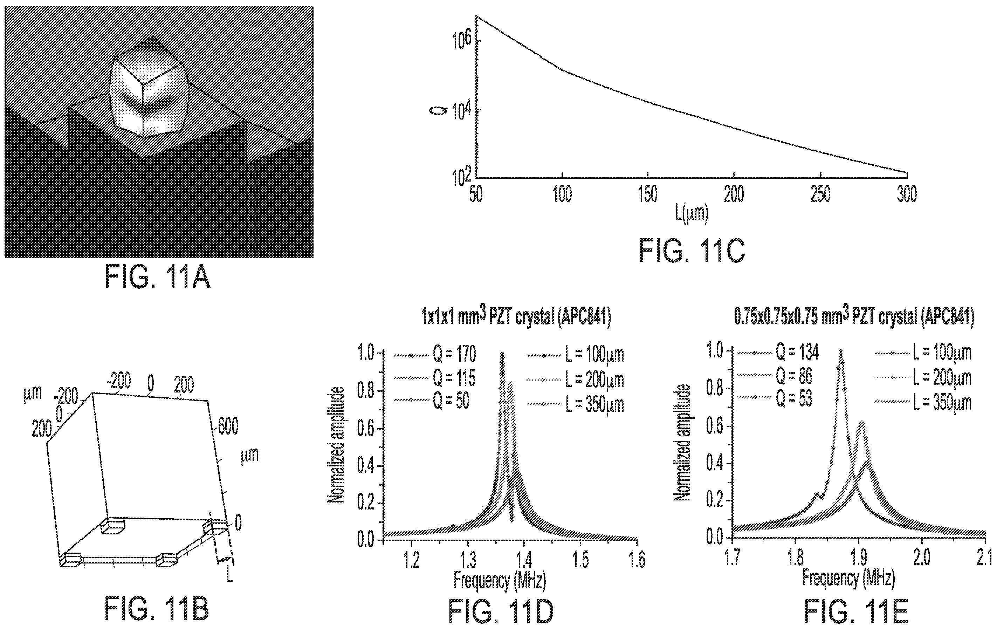

mm³ piezoelectric crystal attached to an ultrasonic wireless implantable device via corner electrodes (as shown in FIG. 11B) having various lengths "L" (and corresponding various mechanical factors (Q)) at a range of frequencies.

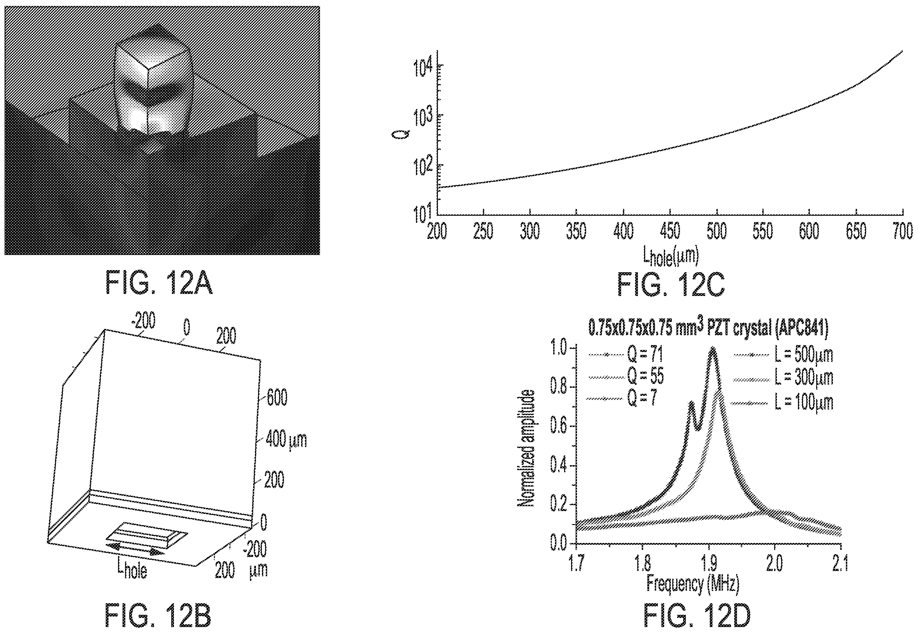

FIG. 12A is a heat map illustrating example deformation in a cubic piezoelectric crystal anchored to the substrate of the ultrasonic wireless implantable device by an electrode or a plurality of electrodes positioned at the edges of one face of the cubic piezoelectric crystal.

FIG. 12B illustrates an example of how an electrode could be positioned at the edges of one face of a cubic piezoelectric crystal to attach the face of the cubic piezoelectric crystal to a substrate.

FIG. 12C is an example graph illustrating a change in mechanical quality factor (Q) of the piezoelectric crystal as the length "L" of the central opening in the electrode as shown in FIG. 12B is increased.

FIG. 12D is an example graph illustrating a normalized amplitude of energy or voltage produced by a 0.75 x 0.75 x 0.75 mm³ piezoelectric crystal attached to an ultrasonic wireless implantable device via an edge electrode (as shown in FIG. 12B) with a central opening having various lengths "L" (and corresponding various mechanical factors (Q)) at a range of frequencies.

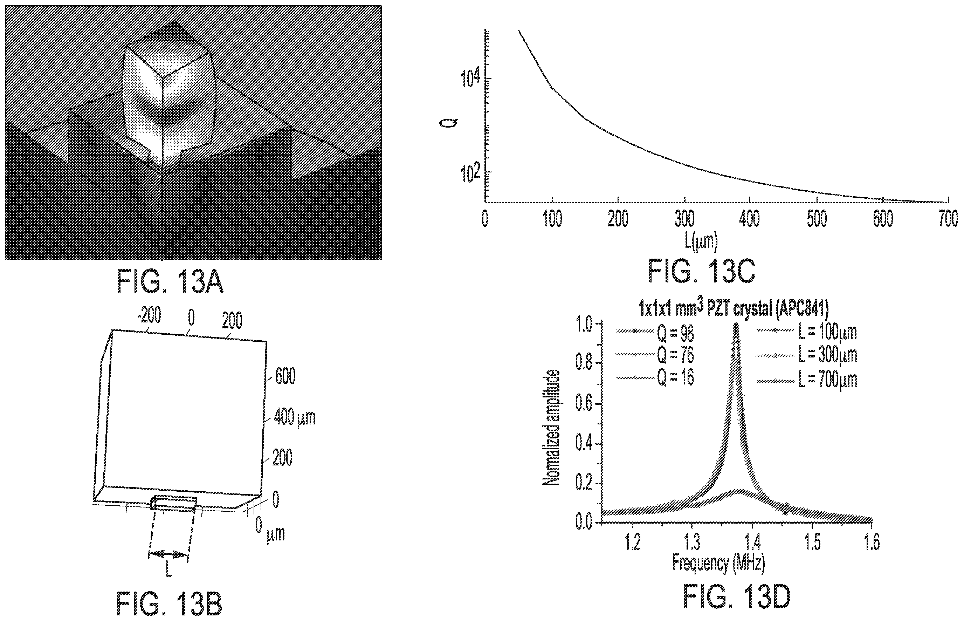

FIG. 13A is a heat map illustrating example deformation in a cubic piezoelectric crystal anchored to the substrate of the ultrasonic wireless implantable device by an electrode or a plurality of electrodes positioned at the center of one face of the cubic piezoelectric crystal.

FIG. 13B illustrates an example of how an electrode could be positioned at the center of one face of a cubic piezoelectric crystal to attach the face of the cubic piezoelectric crystal to a substrate.

FIG. 13C is an example graph illustrating a change in mechanical quality factor (Q) of the piezoelectric crystal as the length "L" of the center electrode (as shown in FIG. 13B) is increased.

FIG. 13D is an example graph illustrating a normalized amplitude of energy or voltage produced by a 1 x 1 x 1 mm³ piezoelectric crystal attached to an ultrasonic wireless implantable device via various center electrodes (as shown in FIG. 13B) having various lengths "L" (and corresponding various mechanical factors (Q)) at a range of frequencies.

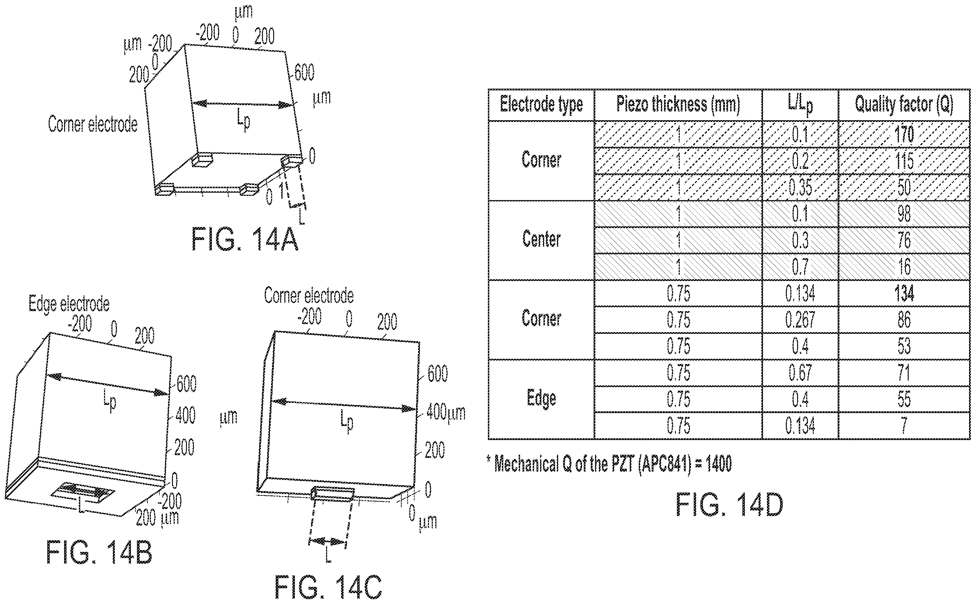

FIG. 14A illustrates an example of how electrodes could be positioned at the corners of one face of a cubic piezoelectric crystal to attach the face of the cubic piezoelectric crystal to a substrate (e.g., as shown in FIG. 11B), with a comparison of the lengths of the electrodes to the length of a face of the cubic piezoelectric crystal.

FIG. 14B illustrates an example of how an electrode could be positioned at the edges of one face of a cubic piezoelectric crystal to attach the face of the cubic piezoelectric crystal to a substrate (e.g., as shown in FIG. 12B), with a comparison of the length of a central opening in the electrode to the length of a face of the cubic piezoelectric crystal.

FIG. 14C illustrates an example of how an electrode could be positioned at the center of one face of a cubic piezoelectric crystal to attach the face of the cubic piezoelectric crystal to a substrate (e.g., as shown in FIG. 13B), with a comparison of the length of the center electrode to the length of a face of the cubic piezoelectric crystal.

FIG. 14D is a chart of example mechanical quality factor (Q) for each type of electrode (corner, edge, and center) for various piezoelectric crystal thicknesses ("$L_p$"), and for various ratios of $L/L_p$.

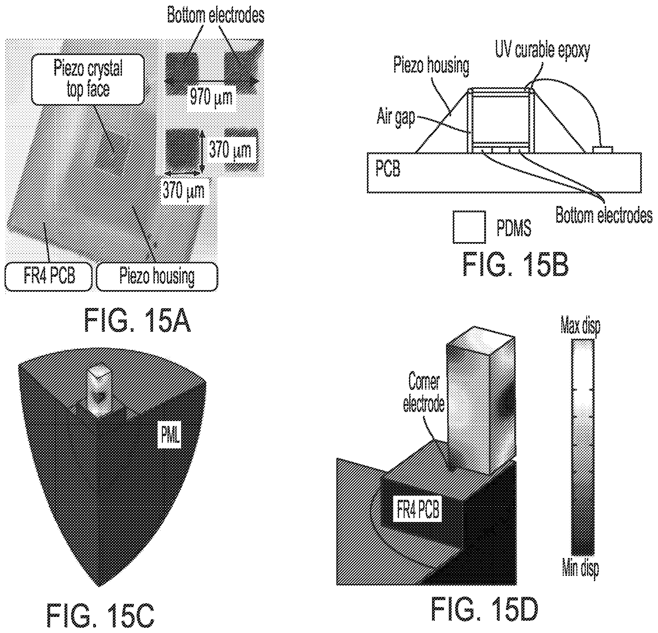

FIG. 15A illustrates the packaged $1 \times 1 \times 1$ mm³ piezoelectric crystal and the electrode configuration that includes four identical square electrodes positioned at the corners of the bottom surface of the piezo.

FIG. 15B illustrates a schematic cross-section of the packaged piezo.

FIG. 15C illustrates the first principal mode shape of the piezo fixed to the corner electrodes on the PCB, simulated using COMSOL with PML to determine wave radiation from the anchor to the substrate and hence anchor loss of the piezo resonator.

FIG. 15D illustrates the quarter of the piezo system shown in FIG. 15C.

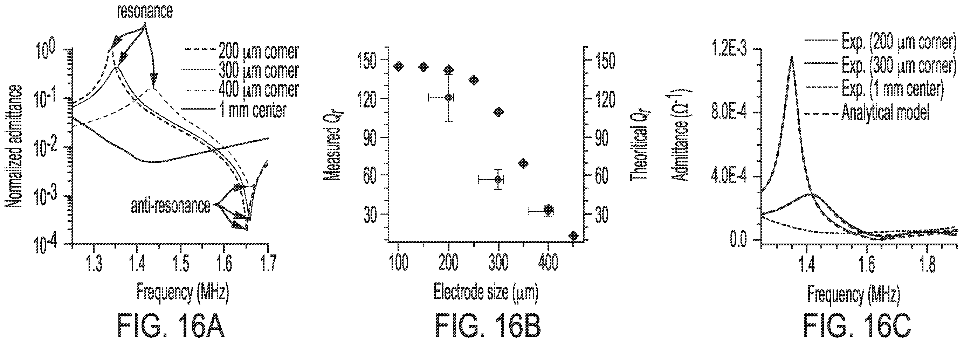

FIGS. 16A-16C illustrate admittance and quality factor ($Q_r$) characterization of the packaged piezocrystals for different electrode dimensions and configurations. In particular, FIG. 16A illustrates measured admittances in air, normalized with respect to the resonance admittance amplitude of the piezo anchored to the $200 \times 200$ $\mu$m corner electrodes. FIG. 16B illustrates measured and theoretical $Q_r$. X error bars: measured min and max electrode size with PCB sample number (n) = 4. Y error bars: $\pm 1$ standard deviation with n = 4. FIG. 16C illustrates measured admittances in water, showing the agreement between experimental data and analytical model predictions.

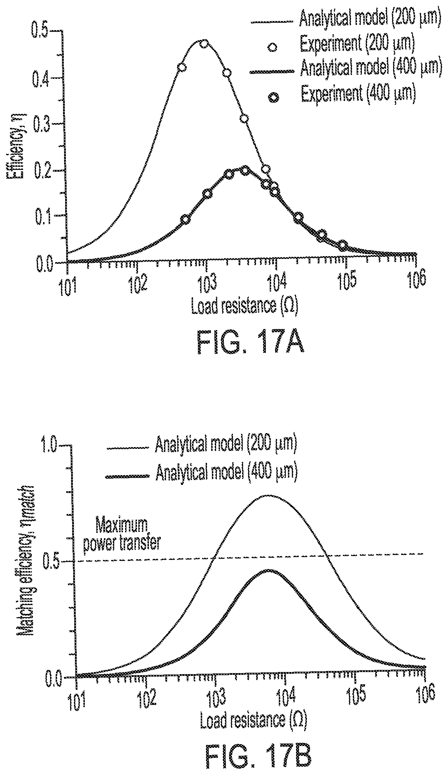

FIG. 17A illustrates efficiency characterization as a function of electrical load resistance at resonance for the piezocrystals packaged on four $200 \times 200$ $\mu$m and $400 \times 400$ $\mu$m corner electrodes for measured and predicted harvesting efficiencies.

FIG. 17B illustrates efficiency characterization as a function of electrical load resistance at resonance for the piezocrystals packaged on four $200 \times 200$ $\mu$m and $400 \times 400$ $\mu$m corner electrodes for predicted matching efficiencies.

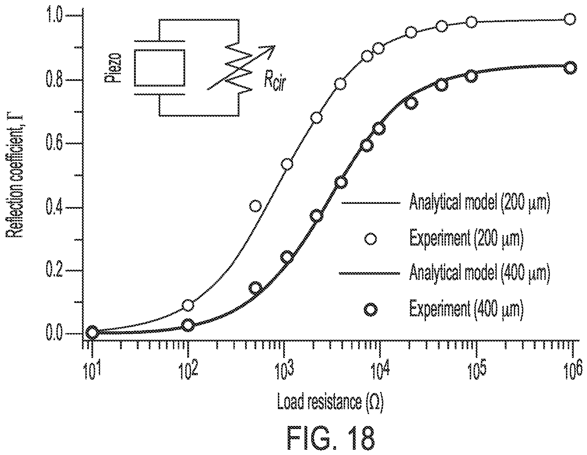

FIG. 18 illustrates measured and predicted acoustic reflection coefficient ($\Gamma$) at resonance versus electrical load resistance for the piezocrystals packaged on four $200 \times 200$ $\mu$m and $400 \times 400$ $\mu$m corner electrodes.

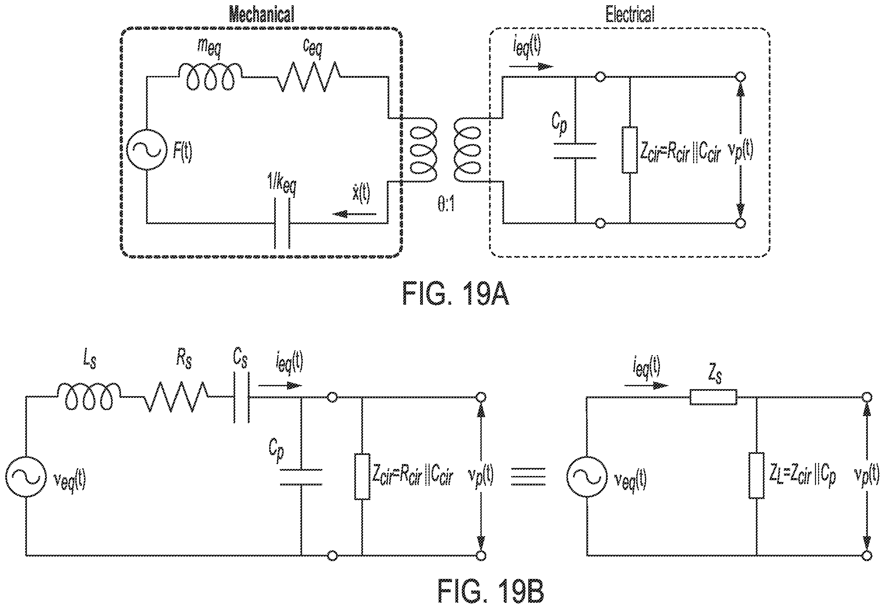

FIG. 19A illustrates an example single degree-of-freedom (DOF) electro-mechanical equivalent circuit of the piezocrystal excited by an external harmonic force, F(t), generated by acoustic pressure waves.

FIG. 19B illustrates an example Thevenin equivalent circuit of the piezocrystal connected to an external electrical load ($Z_{cir}$) for power generation.

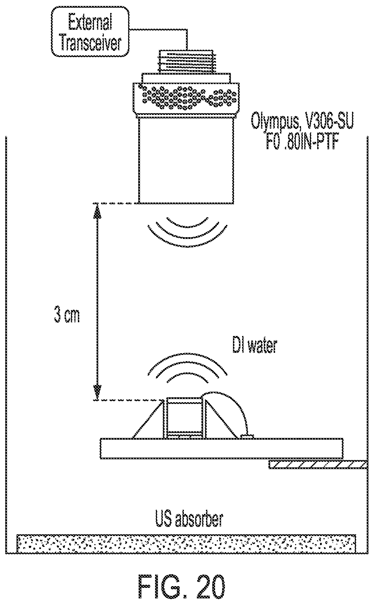

FIG. 20 illustrates a water tank setup used for harvesting efficiency and backscatter characterization of packaged piezocrystals, a 12.7 mm diameter, 2.25 MHz single-element external ultrasound transducer with a focal depth of ~2 cm (V306-SU-F0.80IN-PTF; Olympus) was used to produce acoustic pressure waves. A hydrophone (HGL-0400; Onda) was used to calibrate the output pressure of the external transducer. In measurements, the distance between the piezo and the external ultrasound transducer was set to 3 cm that is far beyond the transducer focal point, resulting in far-field operation. An ultrasound absorber was placed to the bottom of the tank to reduce acoustic reflections from the bottom surface during measurements.

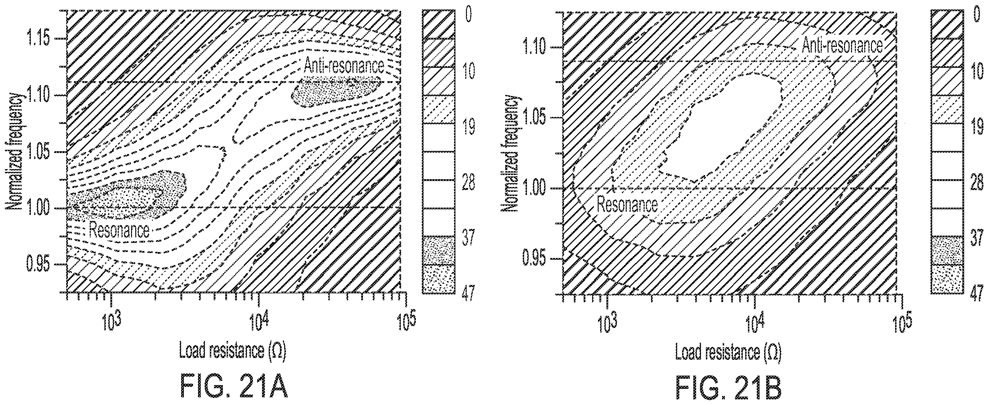

FIG. 21A illustrates measured harvesting efficiencies for piezocrystals packaged on four $200 \times 200$ $\mu$m$^2$ corner electrodes versus electrical load resistance and normalized excitation frequency.

FIG. 21B illustrates measured harvesting efficiencies for piezocrystals packaged on four $400 \times 400$ $\mu$m$^2$ corner electrodes versus electrical load resistance and normalized excitation frequency.

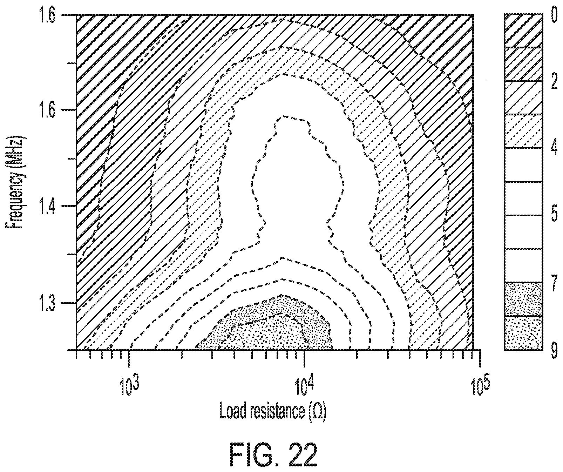

FIG. 22 illustrates the measured harvesting efficiency for a piezocrystal packaged on a $1 \times 1$ mm$^2$ single electrode versus electrical load resistance and excitation frequency.

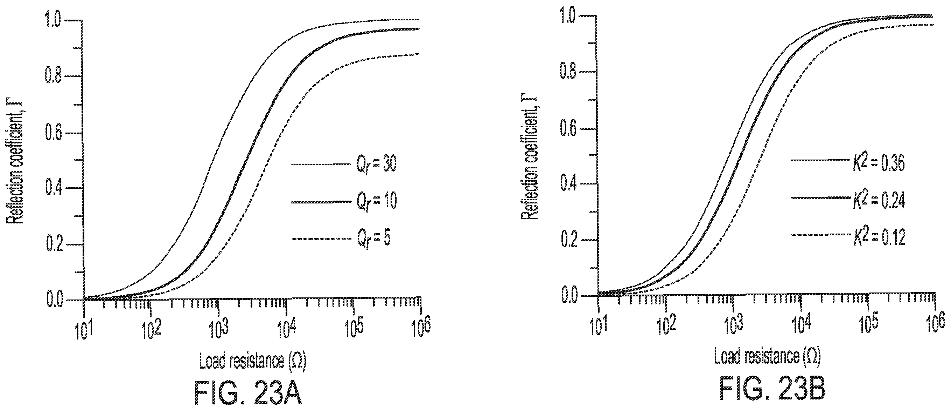

FIG. 23A illustrates acoustic reflection coefficient ($\Gamma$) versus electrical load resistance obtained at resonance by using the parameters of the piezo with 400 $\mu$m anchors for different values of $Q_r$

FIG. 23B illustrates acoustic reflection coefficient ($\Gamma$) versus electrical load resistance obtained at resonance by using the parameters of the piezo with 400 $\mu$m anchors for different values of $\kappa^2$.

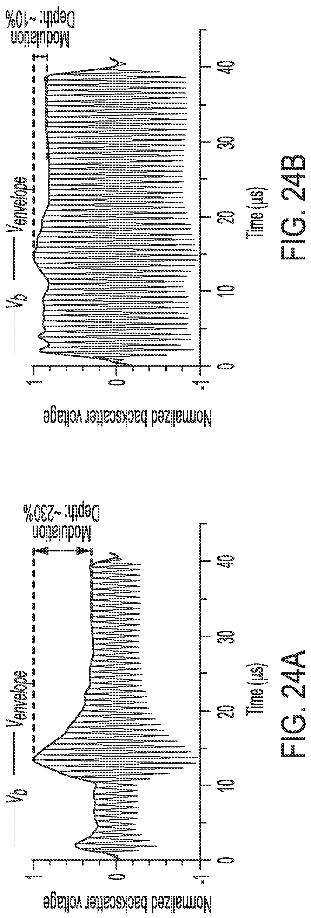

FIG. 24A illustrates normalized backscatter signals recorded from piezocrystals anchored to ($200 \times 200$ $\mu$m corner electrodes, showing backscatter amplitude modulation achieved by switching the electrical load resistance, connected across the piezo, between 500 $\Omega$ and 100 k$\Omega$ during operation.

FIG. 24B illustrates normalized backscatter signals recorded from piezocrystals anchored to ($400 \times 400$ $\mu$m corner electrodes, showing backscatter amplitude modulation achieved by switching the electrical load resistance, connected across the piezo, between 500 $\Omega$ and 100 k$\Omega$ during operation.

## DETAILED DESCRIPTION

Overview

The miniaturization of medical implants to sub-mm dimensions can lead to reduced tissue damage and reduced long-term impact of the implant on surrounding tissue. A growing body of bioelectronics research has focused on developing implantable wireless systems based on sub-mm-sized implants with wide applicability in tissue for both therapies and diagnostics. Most wireless systems rely on electromagnetic (EM) waves for wireless power and data transfer; however, coupling EM waves to sub-mm implants becomes severely inefficient within tissue. Ultrasound has recently emerged as an attractive alternative to EM, to efficiently power and communicate with tiny (sub-mm3) implants deep in tissue (>5 cm).

Ultrasound-based systems employ an implant and an external transceiver outside the body; the external transceiver contains a piezoelectric transducer driven by electronics. This transducer establishes an acoustic link to the implant. The implant consists of at least a piezocrystal and an electrical component, such as an integrated circuit; the piezo harvests acoustic energy and allows for encoding information in the acoustic waves reflected from itself (a process called "backscatter modulation"). The use of backscatter modulation to transmit uplink data from inside to the outside of the body does not require active power to initiate data transmission, potentially eliminating the need for an external capacitor, thereby enabling the design of ultra-small implants.

Recently, many researchers in the bioelectronics field have focused on interrogating these devices implanted in deep areas of the body. A key challenge to operate these devices at centimeter-scale depths in the body is to deliver enough ultrasound energy to an implant without exceeding FDA-regulated safe power limits. Moreover, the size of ultrasonic implants relying on backscatter modulation is limited by the piezo as miniaturization can degrade both power transfer efficiency and data transfer reliability of the acoustic link in tissue. A common approach to this problem is to optimize the system components, such as piezo geometry, piezo material properties, and the size and focusing ability of the external transducer.

A way to overcome these challenges is to maximize acoustic-to-electrical power conversion efficiency of the piezoelectric crystal. Existing ultrasonic implantable devices suffer from low acoustic-to-electrical power conversion efficiency of the piezoelectric crystal which is mainly limited by mechanical quality factor of the piezoelectric crystal, as the power conversion efficiency of the piezoelectric crystal is directly proportional to its mechanical quality factor (Q). Generally speaking, the piezoelectric crystal needs to have very low energy loss to achieve a high mechanical quality factor.

One source of energy loss in a piezoelectric crystal is "anchor loss" - a loss in vibration/oscillation energy generated by the piezoelectric crystal due to "anchoring" the piezoelectric crystal to the substrate to which it is attached.

The present disclosure provides techniques for attaching a piezoelectric crystal to a substrate of an ultrasonic wireless implantable device in a manner that reduces anchor loss. In particular, the present application provides a solution that is based on the packaging of the implant piezo. The approach provided herein relies on positioning the anchors on the implant package at the piezo regions, where the displacement of a vibrational mode of interest is nearly zero. This approach enables reducing anchor loss that causes energy loss through the substrate, maximizing the piezo quality factor. The approach provided herein also improves system coupling ($\kappa 2$). A piezo with higher Q and $\kappa 2$ achieves higher power harvesting efficiency and backscatter amplitude modulation depth at resonance, maximizing system acoustic power and data link efficiency, thereby enabling the miniaturization of ultrasonic implants.

A first study was performed investigating anchor loss causing energy loss through the substrate where the piezoelectric crystal is attached. Specifically, the effects of substrate characteristics - substrate material with different stiffness and thicknesses and attachment configuration of the piezoelectric crystal and substrate - were investigated in the study. Initial study results demonstrate that anchor loss can be significantly reduced by using a thin substrate with low stiffness and small electrodes attaching the piezoelectric crystal to the substrate. In particular, anchor loss can be reduced when these electrodes are positioned to align with the corners of the piezoelectric crystal, the edges of the piezoelectric crystal, or the center of the piezoelectric crystal. Furthermore, anchor loss can be reduced when these electrodes are small compared to the size of the face of the piezoelectric crystal to which they are attached.

A second study was performed investigating piezocrystals assembled with different size anchors. The results of the study indicate that reducing the anchor size decreases anchor loss and thus improves piezo quality factor (Q). The results also show that this method improves system electromechanical coupling. A strongly coupled, high-Q piezo is demonstrated to achieve significantly higher $\eta$ and superior data transfer capability at resonance, ensuring a robust acoustic link that provides a path towards the additional miniaturization of ultrasonic implants.

Definitions

As used herein, "ultrasonic wireless implantable devices" may refer to devices as disclosed by any of the following documents, each of which are incorporated by reference in their entirety: U.S. Patent Application Publication No. 2019/0150883 entitled "IMPLANTS USING ULTRASONIC BACKSCATTER FOR DETECTING ELECTROPHYSIOLOGICAL SIGNALS;" U.S. Patent Application Publication No. 2019/0150882 entitled "IMPLANTS USING ULTRASONIC BACKSCATTER FOR SENSING ELECTRICAL IMPEDANCE OF TISSUE;" U.S. Patent Application Publication No. 2019/0150884 entitled "IMPLANTS USING ULTRASONIC WAVES FOR STIMULATING TISSUE;" U.S Patent No. 10,300,310 entitled "IMPLANTS USING ULTRASONIC BACKSCATTER FOR SENSING PHYSIOLOGICAL CONDITIONS;" U.S Patent No. 10,300,309 entitled "IMPLANTS USING ULTRASONIC BACKSCATTER FOR SENSING PHYSIOLOGICAL CONDITIONS;" U.S. Patent Application Publication No. 2019/0150881 entitled "IMPLANTS USING ULTRASONIC BACKSCATTER FOR RADIATION DETECTION AND ONCOLOGY;" and U.S Patent No. 10,118,054 entitled "IMPLANTS USING ULTRASONIC BACKSCATTER FOR SENSING PHYSIOLOGICAL CONDITIONS."

As used herein, the singular forms "a," "an," and "the" include the plural reference unless the context clearly dictates otherwise.

Reference to "about" a value or parameter herein includes (and describes) variations that are directed to that value or parameter per se. For example, description referring to "about X" includes description of "X".

The term "miniaturized" refers to any material or component about 5 millimeters or less (such as about 4 mm or less, about 3 mm or less, about 2 mm or less, about 1 mm or less, or about 0.5 mm or less) in length in the longest dimension. In certain embodiments, a "miniaturized" material or component has a longest dimension of about 0.1 mm to about 5 mm (such as about 0.2 mm to about 5 mm, about 0.5 mm to about 5 mm, about 1 mm to about 5 mm, about 2 mm to about 5 mm, about 3 mm to about 5 mm, or about 4 mm to about 5 mm) in length. "Miniaturized" can also refer to any material or component with a volume of about 5 mm or less (such as about 4 mm 3 or less, 3 mm 3 or less, 2 mm 3 or less, or 1 mm 3 or less). In certain embodiments, a "miniaturized" material or component has a volume of about 0.5 mm to about 5 mm 3 , about 1 mm 3 to about 5 mm 3 , about 2 mm 3 to about 5 mm 3 , about 3 mm 3 to about 5 mm 3 , or about 4 mm 3 to about 5 mm 3.

A "piezoelectric transducer" is a type of ultrasonic transceiver comprising piezoelectric material. The piezoelectric material may be a crystal, a ceramic, a polymer, or any other natural or synthetic piezoelectric material.

A "non-responsive" ultrasonic wave is an ultrasonic wave with a reflectivity independent of a detected signal. A "non-responsive reflector" is a component of an implantable device that reflects ultrasonic waves such that the reflected waveform is independent of the detected signal.

The term "subject" refers to an animal.

It is understood that aspects and variations of the invention described herein include "consisting" and/or "consisting essentially of" aspects and variations.

Where a range of values is provided, it is to be understood that each intervening value between the upper and lower limit of that range, and any other stated or intervening value in that stated range, is encompassed within the scope of the present disclosure. Where the stated range includes upper or lower limits, ranges excluding either of those included limits are also included in the present disclosure.

It is to be understood that one, some or all of the properties of the various embodiments described herein may be combined to form other embodiments of the present invention. The section headings used herein are for organizational purposes only and are not to be construed as limiting the subject matter described.

Features and preferences described above in relation to "embodiments" are distinct preferences and are not limited only to that particular embodiment; they may be freely combined with features from other embodiments, where technically feasible, and may form preferred combinations of features.

The description is presented to enable one of ordinary skill in the art to make and use the invention and is provided in the context of a patent application and its requirements. Various modifications to the described embodiments will be readily apparent to those persons skilled in the art and the generic principles herein may be applied to other embodiments. Thus, the present invention is not intended to be limited to the embodiment shown but is to be accorded the widest scope consistent with the principles and features described herein. Further, sectional headings are provide for organizational purposes and are not to be considered limiting.

Neural Dust System

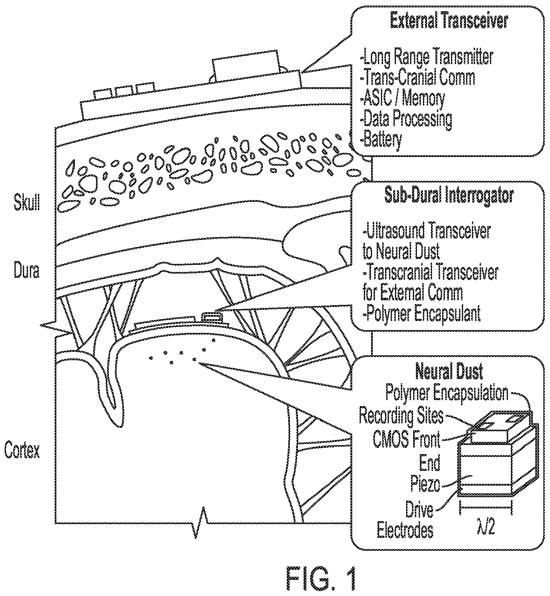

FIG. 1 is a schematic of a neural dust system, including an external transceiver, a subdural interrogator, and a neural dust mote.

Interrogator

The interrogator can wirelessly communicate with one or more implantable devices using ultrasonic waves, which are used to power and/or operate the implantable device. The ultrasonic waves emitted by the interrogator can encode a trigger signal, which signals the implantable device to emit an electrical pulse. The interrogator includes one or more ultrasonic transducers, which can operate as an ultrasonic transmitter and/or an ultrasonic receiver (or as a transceiver, which can be configured to alternatively transmit or receive the ultrasonic waves). The one or more transducers can be arranged as a transducer array, and the interrogator can optionally include one or more transducer arrays. In some embodiments, transducers in the array can have regular spacing, irregular spacing, or be sparsely placed. In some embodiments the array is flexible. In some embodiments the array is planar, and in some embodiments the array is non-planar. In some embodiments, the ultrasound transmitting function is separated from the ultrasound receiving function on separate devices. That is, optionally, the interrogator comprises a first device that transmits ultrasonic waves to the implantable device, and a second device that receives ultrasonic backscatter from the implantable device.

In some embodiments, the interrogator can receive ultrasonic backscatter from an implantable device, such an implantable device configured to detect an electrophysiological voltage and emit ultrasonic backscatter which encodes information indicative of the detected electrophysiological voltage signal. In some embodiments, the trigger signal encoded by the ultrasonic waves emitted from the interrogator and received by the implantable device configured to emit an electrical pulse is transmitted in response to a received ultrasonic backscatter encoding information regarding the detected electrophysiological signal.

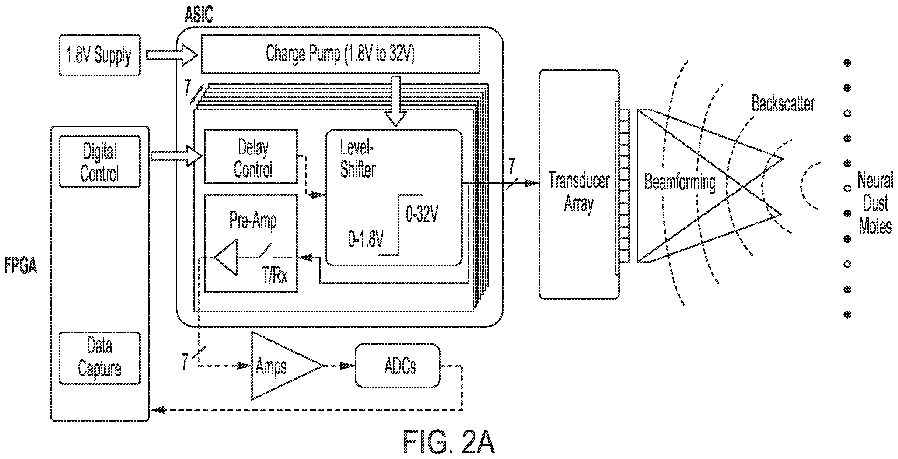

An exemplary interrogator is shown in FIG. 2A. The illustrated interrogator shows a transducer array with a plurality of ultrasonic transducers. In some embodiments, the transducer array includes 1 or more, 2 or more, 3 or more, 5 or more, 7 or more, 10 or more, 15 or more, 20 or more, 25 or more, 50 or more, 100 or more 250 or more, 500 or more, 1000 or more, 2500 or more, 5000 or more, or 10,000 or more transducers. In some embodiments, the transducer array includes 100,000 or fewer, 50,000 or fewer, 25,000 or fewer, 10,000 or fewer, 5000 or fewer, 2500 or fewer, 1000 or fewer, 500 or fewer, 200 or fewer, 150 or fewer, 100 or fewer, 90 or fewer, 80 or fewer, 70 or fewer, 60 or fewer, 50 or fewer, 40 or fewer, 30 or fewer, 25 or fewer, 20 or fewer, 15 or fewer, 10 or fewer, 7 or fewer or 5 or fewer transducers. The transducer array can be, for example a chip comprising 50 or more ultrasonic transducer pixels. The interrogator shown in FIG. 2A illustrates a single transducer array; however the interrogator can include 1 or more, 2 or more, or 3 or more separate arrays. In some embodiments, the interrogator includes 10 or fewer transducer arrays (such as 9, 8, 7, 6, 5, 4, 3, 2, or 1 transducer arrays). The separate arrays, for example, can be placed at different points of a subject, and can communicate to the same or different implantable devices. In some embodiments, the arrays are located on opposite sides of an implantable device. The interrogator can include an ASIC, which includes a channel for each transducer in the transducer array. In some embodiments, the channel includes a switch (indicated in FIG. 2A by "T/Rx"). The switch can alternatively configure the transducer connected to the channel to transmit ultrasonic waves or receive ultrasonic waves. The switch can isolate the ultrasound receiving circuit from the higher voltage ultrasound transmitting circuit. In some embodiments, the transducer connected to the channel is configured only to receive or only to transmit ultrasonic waves, and the switch is optionally omitted from the channel. The channel can include a delay control, which operates to control the transmitted ultrasonic waves. The delay control can control, for example, the phase shift, time delay, pulse frequency and/or wave shape (including amplitude and wavelength). The delay control can be connected to a level shifter, which shifts input pulses from the delay control to a higher voltage used by the transducer to transmit the ultrasonic waves. In some embodiments, the data representing the wave shape and frequency for each channel can be stored in a 'wave table'. This allows the transmit waveform on each channel to be different. Then, delay control and level shifters can be used to 'stream' out this data to the actual transmit signals to the transducer array. In some embodiments, the transmit waveform for each channel can be produced directly by a high-speed serial output of a microcontroller or other digital system and sent to the transducer element through a level shifter or high- voltage amplifier. In some embodiments, the ASIC includes a charge pump (illustrated in FIG. 2A) to convert a first voltage supplied to the ASIC to a higher second voltage, which is applied to the channel. The channels can be controlled by a controller, such as a digital controller, which operates the delay control. In the ultrasound receiving circuit, the received ultrasonic waves are converted to current by the transducers (set in a receiving mode), which is transmitted to a data capture circuit. In some embodiments, an amplifier, an analog-to-digital converter (ADC), a variable-gain-amplifier, or a time-gain- controlled variable-gain-amplifier which compensates for tissue loss, and/or a band pass filter is included in the receiving circuit. The ASIC can draw power from a power supply, such as a battery (which is preferred for a wearable embodiment of the interrogator). In the embodiment illustrated in FIG. 2A, a 1.8V supply is provided to the ASIC, which is increased by the charge pump to 32V, although any suitable voltage can be used. In some embodiments, the interrogator includes a processor and or a non-transitory computer readable memory. In some embodiments, the channel described above does not include a T/Rx switch but instead contains independent Tx (transmit) and Rx (receive) with a high- voltage Rx (receiver circuit) in the form of a low noise amplifier with good saturation recovery. In some embodiments, the T/Rx circuit includes a circulator. In some embodiments, the transducer array contains more transducer elements than processing channels in the interrogator transmit /receive circuitry, with a multiplexer choosing different sets of transmitting elements for each pulse. For example, 64 transmit receive channels connected via a 3: 1 multiplexer to 192 physical transducer elements - with only 64 transducer elements active on a given pulse.

FIG. 2B illustrates another embodiment of interrogator. As shown in FIG. 2B, the interrogator includes one or more transducers 202. Each transducer 202 is connected to a transmitter/receiver switch 204, which can alternatively configure the transducer to transmit or receive ultrasonic waves. The transmitter/receiver switch is connected to a processor 206 (such as a central processing unit (CPU), a custom dedicated processor ASIC, a field programmable gate array (FPGA), microcontroller unit (MCU), or a graphics processing unit (GPU)). In some embodiments, the interrogator further includes an analog-digital converter (ADC) or digital-to-analog converter (DAC). The interrogator can also include a user interface (such as a display, one or more buttons to control the interrogator, etc.), a memory, a power supply (such as a battery), and/or an input/output port (which may be wired or wireless).

In some embodiments, the interrogator is implantable. An implanted interrogator may be preferred when the implantable devices are implanted in a region blocked by a barrier that does not easily transmit ultrasonic waves. For example, the interrogator can be implanted subcranially, either subdurally or supradurally. A subcranial interrogator can communicate with implantable devices that are implanted in the brain. Since ultrasonic waves are impeded by the skull, the implanted subcranial interrogator allows for communication with the implantable devices implanted in the brain. In another example, an implantable interrogator can be implanted as part of, behind or within another implanted device, such as a bone plate. The implanted interrogator can communicate with an external device, for example by EM or RF signals.

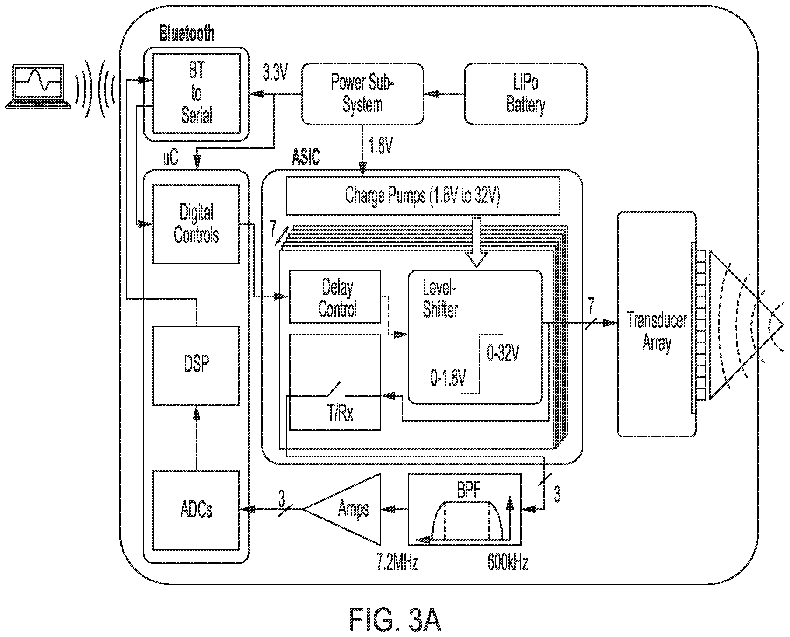

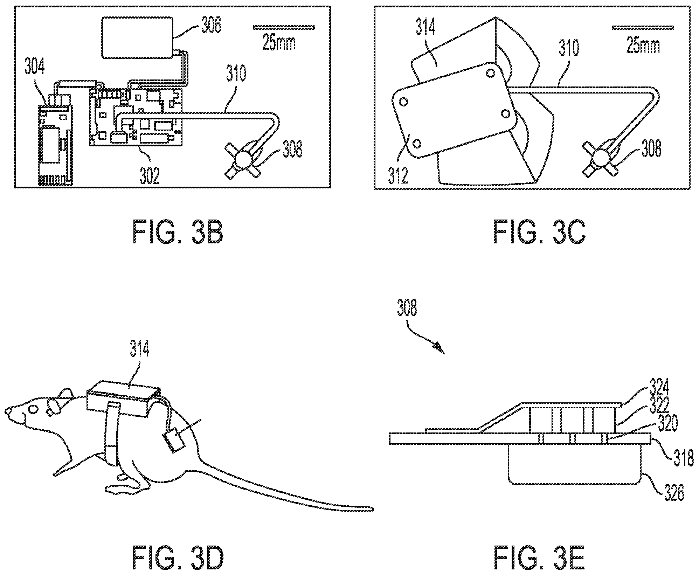

In some embodiments, the interrogator is external (i.e., not implanted). By way of example, the external interrogator can be a wearable, which may be fixed to the body by a strap or adhesive. In another example, the external interrogator can be a wand, which may be held by a user (such as a healthcare professional). In some embodiments, the interrogator can be held to the body via suture, simple surface tension, a clothing-based fixation device such as a cloth wrap, a sleeve, an elastic band, or by sub-cutaneous fixation. The transducer or transducer array of the interrogator may be positioned separately from the rest of the transducer. For example, the transducer array can be fixed to the skin of a subject at a first location (such as proximal to one or more implanted devices), and the rest of the interrogator may be located at a second location, with a wire tethering the transducer or transducer array to the rest of the interrogator. FIG. 3A-E shows an example of a wearable external interrogator. FIG. 3A shows a block diagram of the interrogator, which includes a transducer array comprising a plurality of transducers, an ASIC comprising a channel for each transducer in the transducer array, a battery (lithium polymer (LiPo) battery, in the illustrated example), and a wireless communication system (such as a Bluetooth system). FIG. 3B illustrates an exploded view of a wearable interrogator, including a printed circuit board (PCB) 302, which includes the ASIC, a wireless communication system 304, a battery 306, an ultrasonic transducer array 308, and a wire 310 tethering the ultrasonic transducer array 308 to the ASIC. FIG. 3C shows the wearable interrogator 312 shown in FIG. 3B with a harness 314, which can be used to attach the interrogator to a subject. FIG. 3D shows the assembled interrogator 316 attached to a subject, with the transducer array 308 attached at a first location, and the rest of the interrogator attached to a second location. FIG. 3E shows a cross-section schematic of an exemplary ultrasonic transducer array 308, which includes a circuit board 318, vias 320 attaching each transducer 322 to the circuit board 318, a metalized polyester film 324, and an absorptive backing layer 326. The metalized polyester film 324 can provide a common ground and acoustic matching for the transducers, while the absorptive backing layer 326 (such as tungsten powder filled polyurethane) can reduce ringing of the individual transducers.

The specific design of the transducer array depends on the desired penetration depth, aperture size, and size of the individual transducers within the array. The Rayleigh distance, R, of the transducer array is computed as:

$$R = \frac{D^2 - \lambda^2}{4\lambda} \approx \frac{D^2}{4\lambda}, D^2 \gg \lambda^2$$

where D is the size of the aperture and $\lambda$ is the wavelength of ultrasound in the propagation medium (i.e., the tissue). As understood in the art, the Rayleigh distance is the distance at which the beam radiated by the array is fully formed. That is, the pressure filed converges to a natural focus at the Rayleigh distance in order to maximize the received power. Therefore, in some embodiments, the implantable device is approximately the same distance from the transducer array as the Rayleigh distance.

The individual transducers in a transducer array can be modulated to control the Rayleigh distance and the position of the beam of ultrasonic waves emitted by the transducer array through a process of beamforming or beam steering. Techniques such as linearly constrained minimum variance (LCMV) beamforming can be used to communicate a plurality of implantable devices with an external ultrasonic transceiver. See, for example, Bertrand et al., Beamforming Approaches for Untethered, Ultrasonic Neural Dust Motes for Cortical Recording: a Simulation Study, IEEE EMBC (Aug. 2014). In some embodiments, beam steering is performed by adjusting the power or phase of the ultrasonic waves emitted by the transducers in an array.

In some embodiments, the interrogator includes one or more of instructions for beam steering ultrasonic waves using one or more transducers, instructions for determining the relative location of one or more implantable devices, instructions for monitoring the relative movement of one or more implantable devices, instructions for recording the relative movement of one or more implantable devices, and instructions for deconvoluting backscatter from a plurality of implantable devices.

Communication Between an Implantable Device and an Interrogator

The implantable device and the interrogator wirelessly communicate with each other using ultrasonic waves. The implantable device receives ultrasonic waves from the interrogator through a miniaturized ultrasonic transducer on the implantable device. Vibrations of the miniaturized ultrasonic transducer on the implantable device generate a voltage across the electric terminals of the transducer and a current flows through the device, including the sensor and/or, if present, the ASIC.

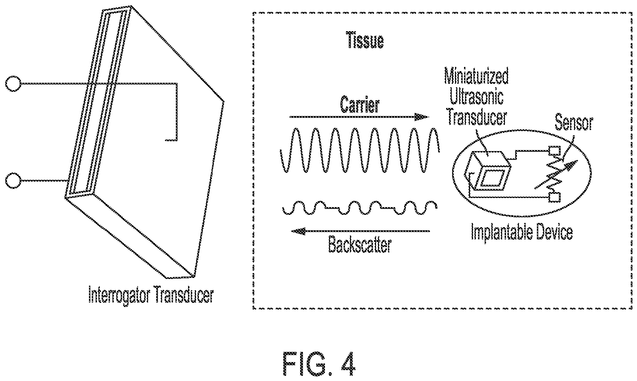

FIG. 4 illustrates an interrogator in communication with an implantable device. The external ultrasonic transceiver emits ultrasonic waves ("carrier waves"), which can pass through tissue. The carrier waves cause mechanical vibrations on the miniaturized ultrasonic transducer (e.g., a miniaturized bulk piezoelectric transducer). A voltage across the miniaturized ultrasonic transducer is generated, which imparts a current flowing through a sensor on the implantable device. In some embodiments, the implantable device includes an ASIC, and current flows from the miniaturized ultrasonic transducer, through the ASIC, to the radiation detector, back to the ASIC, and returns to the miniaturized ultrasonic transducer. The current flowing through the miniaturized ultrasonic transducer causes the transducer on the implantable device to emit backscatter ultrasonic waves.

Communication between the interrogator and the implantable device can use a pulse-echo method of transmitting and receiving ultrasonic waves. In the pulse-echo method, the interrogator transmits a series of interrogation pulses at a predetermined frequency, and then receives backscatter echoes from the implanted device. In some embodiments, the pulses are about 200 nanoseconds (ns) to about 1000 ns in length (such as about 300 ns to about 800 ns in length, about 400 ns to about 600 ns in length, or about 540 ns in length). In some embodiments, the pulses are about 100 ns or more in length (such as about 150 ns or more, 200 ns or more, 300 ns or more, 400 ns or more, 500 ns or more, 540 ns or more, 600 ns or more, 700 ns or more, 800 ns or more, 900 ns or more, 1000 ns or more, 1200 ns or more, or 1500 ns or more in length). In some embodiments, the pulses are about 2000 ns or less in length (such as about 1500 ns or less, 1200 ns or less, 1000 ns or less, 900 ns or less, 800 ns or less, 700 ns or less, 600 ns or less, 500 ns or less, 400 ns or less, 300 ns or less, 200 ns or less, or 150 ns or less in length). In some embodiments, the pulses are separated by a dwell time. In some embodiments, the dwell time is about 100 ns or more in length (such as about 150 ns or more, 200 ns or more, 300 ns or more, 400 ns or more, 500 ns or more, 540 ns or more, 600 ns or more, 700 ns or more, 800 ns or more, 900 ns or more, 1000 ns or more, 1200 ns or more, or 1500 ns or more in length). In some embodiments, the dwell time is about 2000 ns or less in length (such as about 1500 ns or less, 1200 ns or less, 1000 ns or less, 900 ns or less, 800 ns or less, 700 ns or less, 600 ns or less, 500 ns or less, 400 ns or less, 300 ns or less, 200 ns or less, or 150 ns or less in length). In some embodiments, the pulses are square, rectangular, triangular, sawtooth, or sinusoidal. In some embodiments, the pulses output can be two-level (GND and POS), three-level (GND, NEG, POS), 5-level, or any other multiple-level (for example, if using 24-bit DAC). In some embodiments, the pulses are continuously transmitted by the interrogator during operation. In some embodiments, when the pulses are continuously transmitted by the interrogator a portion of the transducers on the interrogator are configured to receive ultrasonic waves and a portion of the transducers on the interrogator are configured to transmit ultrasonic waves. Transducers configured to receive ultrasonic waves and transducers configured to transmit ultrasonic waves can be on the same transducer array or on different transducer arrays of the interrogator. In some embodiments, a transducer on the interrogator can be configured to alternatively transmit or receive the ultrasonic waves. For example, a transducer can cycle between transmitting one or more pulses and a pause period. The transducer is configured to transmit the ultrasonic waves when transmitting the one or more pulses, and can then switch to a receiving mode during the pause period. In some embodiments, the one or more pulses in the cycle includes about 1 to about 10 pulses (such as about 2 to about 8, or about 4 to about 7, or about 6) pulses of ultrasonic waves in any given cycle. In some embodiments, the one or more pulses in the cycle includes about 1 or more, 2 or more, 4 or more, 6 or more, 8 or more, or 10 or more pulses of ultrasonic waves in any given cycle. In some embodiments, the one or more pulses in the cycle includes about 20 or fewer, about 15 or fewer, about 10 or fewer, about 8 or fewer, or about 6 or fewer pulses in the cycle. The pulse cycle can be regularly repeated, for example every about 50 microseconds ([is) to about 300 [is (such as about every 75 [is to about 200 [is, or every about 100 [is) during operation. In some embodiments, the cycle is reaped every 50 [is or longer, every 100 [is or longer, every 150 [is or longer, every 200 [is or longer, every 250 [is or longer, or every 300 [is or longer. In some embodiments, the cycle is repeated every 300 [is or sooner, every 250 [is or sooner, every 200 [is or sooner, every 150 [is or sooner, or every 100 [is or sooner. The cycle frequency can set, for example, based on the distance between the interrogator and the implantable device and/or the speed at which the transducer can toggle between the transmitting and receiving modes.

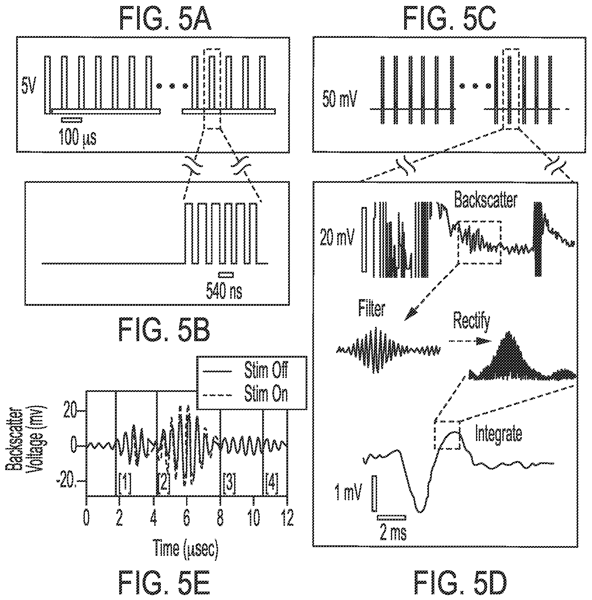

FIG. 5 illustrates cycled pulse-echo ultrasonic communication between the interrogator and the implantable device. FIG. 5A shows a series of pulse cycles with a frequency of every 100 microseconds. During the transmission of the pulses, the transducers in the array are configured to transmit the ultrasonic waves. After the pulses are transmitted, the transducers are configured to receive backscattered ultrasonic waves. FIG. 5B shows a zoom-in view of a cycle, which shows six pulses of ultrasonic waves, with a frequency of every 540 nanoseconds. Backscattered ultrasonic waves detected by the interrogator are shown in FIG. 5C, with a zoom-in view of a single pulse shown in FIG. 5D. As shown in FIG. 5D, the ultrasonic backscatter received from the implantable device can be analyzed, which may include filtering (for example, to remove the wave decay) the backscattered waves, rectifying the backscattered waves, and integrating the waves to determine the data encoded by the waves. In some embodiments, the backscatter waves are analyzed using a machine learning algorithm. FIG. 5E shows a zoomed in version of the filtered backscattered waves. The backscatter wave shown in FIG. 5E includes four distinct regions corresponding to reflections arising from mechanical boundaries: (1) reflection from the biocompatible material that encapsulates the implantable device; (2) reflection from the top surface of the miniaturized ultrasonic transducer; (3) reflection from the boundary between the printed circuit board and the miniaturized ultrasonic transducer; and (4) reflection from the back of the printed circuit board. The amplitude of the backscatter waves reflected from the surface of the miniaturized transducer changed as a function of changes in impedance of the current returning to the miniaturized ultrasonic transducer, and can be referred to as the "responsive backscatter" since this region of the backscatter encodes information relating to the sensed physiological condition. The other regions of the ultrasonic backscatter can be referred to as "non-responsive backscatter," and are useful in determining the position of the implantable device, movement of the implantable device, and/or temperature changes proximal to the implantable device, as explained below. In some embodiments, the device further comprises a non-responsive reflector. In some embodiments, the non-responsive reflector is a cube. In some embodiments, the non-responsive reflector comprises silicon. In some embodiments, the non-responsive reflector is a surface of rigid material. The non-responsive reflector is attached to the implantable device but electrically isolated, and can reflect ultrasonic waves that are not responsive to changes in sensor or ASIC impedance.

The frequency of the ultrasonic waves transmitted by the transducer can be set depending on the drive frequency or resonant frequency of the miniaturized ultrasonic transducer on the implantable device. In some embodiments, the miniaturized ultrasonic transducers are broad-band devices. In some embodiments, the miniaturized ultrasonic transducers are narrowband. For example, in some embodiments the frequency of the pulses is within about 20% or less, within about 15% or less, within about 10% or less, within about 5% or less of the resonant frequency of the miniaturized ultrasonic transducer. In some embodiments, the pulses are set to a frequency about the resonant frequency of the miniaturized ultrasonic transducer. In some embodiments, the frequency of the ultrasonic waves is between about 100 kHz and about 100 MHz (such as between about 100 kHz and about 200 kHz, between about 200 kHz and about 500 kHz, between about 500 kHz and about 1 MHz, between about 1 MHz and about 5 MHz, between about 5 MHz and about 10 MHz, between about 10 MHz and about 25 MHz, between about 25 MHz and about 50 MHz, or between about 50 MHz and about 100 MHz). In some embodiments, the frequency of the ultrasonic waves is about 100 kHz or higher, about 200 kHz or higher, about 500 kHz or higher, about 1 MHz or higher, about 5 MHz or higher, about 10 MHz or higher, about 25 MHz or higher, or about 50 MHz or higher. In some embodiments, the frequency of the ultrasonic waves is about 100 MHz or lower, about 50 MHz or lower, about 25 MHz or lower, about 10 MHz or lower, about 5 MHz or lower, about 1 MHz or lower, about 500 kHz or lower, or about 200 kHz or lower. Higher frequency allows for a smaller miniaturized ultrasonic transducer on the implantable device. However, higher frequency also limits the depth of communication between the ultrasonic transducer and the implantable device. In some embodiments, the implantable device and the ultrasonic transducer are separated by about 0.1 cm to about 15 cm (such as about 0.5 cm to about 10 cm, or about 1 cm to about 5 cm). In some embodiments, the implantable device and the ultrasonic transducer are separated by about 0.1 cm or more, about 0.2 cm or more, about 0.5 cm or more, about 1 cm or more, about 2.5 cm or more, about 5 cm or more, about 10 cm or more, or about 15 cm or more. In some embodiments, the implantable device and the ultrasonic transducer are separated by about 20 cm or less, about 15 cm or less, about 10 cm or less, about 5 cm or less, about 2.5 cm or less, about 1 cm or less, or about 0.5 cm or less.

In some embodiments, the backscattered ultrasound is digitized by the implantable device. For example, the implantable device can include an oscilloscope or analog-to-digital converter (ADC) and/or a memory, which can digitally encode information in current (or impedance) fluctuations. The digitized current fluctuations, which reflect data sensed by the sensor, are received by the ultrasonic transducer, which then transmits digitized acoustic waves. The digitized data can compress the analog data, for example by using singular value decomposition (SVD) and least squares-based compression. In some embodiments, the compression is performed by a correlator or pattern detection algorithm. The backscatter signal may go through a series of non-linear transformation, such as 4th order Butterworth bandpass filter rectification integration of backscatter regions to generate a reconstruction data point at a single time instance. Such transformations can be done either in hardware (i.e., hard-coded) or in software.

In some embodiments, the digitized data can include a unique identifier. The unique identifier can be useful, for example, in a system comprising a plurality of implantable devices and/or an implantable device comprising a plurality of electrode pairs. For example, the unique identifier can identify the implantable device of signal origin when from a plurality of implantable devices. In some embodiments, an implantable device comprises a plurality of electrode pairs, which may simultaneously or alternatively receive electrophysiological signals that are detected by a single implantable device. Different pairs of electrodes, for example, can be configured to detect electrophysiological signals in different tissues (e.g., different nerves or different muscles) or in different regions of the same tissue. The digitized circuit can encode a unique identifier to identify which electrode pairs detected the electrophysiological signal.

In some embodiments, the digitized signal compresses the size of the analog signal. The decreased size of the digitized signal can allow for more efficient reporting of detected electrophysiological signals encoded in the ultrasonic backscatter. This can be useful, for example, when an implantable device includes a plurality of electrode pairs that simultaneously or approximately simultaneously detect an electrophysiological signal. By compressing the size of the electrophysiological signal through digitization, potentially overlapping signals can be accurately transmitted.

In some embodiments, an interrogator communicates with a plurality of implantable devices. This can be performed, for example, using multiple-input, multiple output (MIMO) system theory. For example, communication between the interrogator and the plurality of implantable devices using time division multiplexing, spatial multiplexing, or frequency multiplexing. In some embodiments, two or more (such as 3, 4, 5, 6, 7, 8, 9, 10 or more, 12 or more, about 15 or more, about 20 or more, about 25 or more, about 50 or more, or about 100 or more) implantable devices communicate with the interrogator. In some embodiments, about 200 or fewer implantable devices (such as about 150 or fewer, about 100 or fewer, about 50 or fewer, about 25 or fewer, about 20 or fewer, about 15 or fewer, about 12 or fewer, or about 10 or fewer implantable devices) are in communication with the interrogator. The interrogator can receive a combined backscatter from the plurality of the implantable devices, which can be deconvoluted, thereby extracting information from each implantable device. In some embodiments, interrogator focuses the ultrasonic waves transmitted from a transducer array to a particular implantable device through beam steering. The interrogator focuses the transmitted ultrasonic waves to a first implantable device, receives backscatter from the first implantable device, focuses transmitted ultrasonic waves to a second implantable device, and receives backscatter from the second implantable device. In some embodiments, the interrogator transmits ultrasonic waves to a plurality of implantable devices, and then receives ultrasonic waves from the plurality of implantable devices.

In some embodiments, the interrogator is used to determine the location or velocity of the implantable device. Velocity can be determined, for example, by determining the position or movement of a device over a period of time. The location of the implantable device can be a relative location, such as the location relative on the transducers on the interrogator. Knowledge of the location or movement of the implantable device allows for knowledge of the precise location of the electrophysiological signal detected in the tissue. By determining the location of the implantable device and associating the location with the detected electrophysiological signal, it is possible to characterize or monitor the tissue at a more localized point. A plurality of transducers on the interrogator, which may be disposed on the same transducer array or two or more different transducer arrays, can collect backscatter ultrasonic waves from an implantable device. Based on the differences between the backscatter waveform arising from the same implantable device and the known location of each transducer, the position of the implantable device can be determined. This can be done, for example by triangulation, or by clustering and maximum likelihood. The differences in the backscatter may be based on responsive backscatter waves, non-responsive backscatter waves, or a combination thereof.

In some embodiments, the interrogator is used to determine the location or velocity of the implantable device. Velocity can be determined, for example, by determining the position or movement of a device over a period of time. The location of the implantable device can be a relative location, such as the location relative on the transducers on the interrogator. Knowledge of the location or movement of the implantable device allows for knowledge of the precise location of the electrophysiological signal detected in the tissue. By determining the location of the implantable device and associating the location with the detected electrophysiological signal, it is possible to characterize or monitor the tissue at a more localized point. A plurality of transducers on the interrogator, which may be disposed on the same transducer array or two or more different transducer arrays, can collect backscatter ultrasonic waves from an implantable device. Based on the differences between the backscatter waveform arising from the same implantable device and the known location of each transducer, the position of the implantable device can be determined. This can be done, for example by triangulation, or by clustering and maximum likelihood. The differences in the backscatter may be based on responsive backscatter waves, non-responsive backscatter waves, or a combination thereof.

In some embodiments, the interrogator is used to track movement of the implantable device. Movement of the implantable device that can be tracked by the interrogator includes lateral and angular movement. Such movement may arise, for example, due to shifting of one or more organs such as the liver, stomach, small or large intestine, kidney, pancreas, gallbladder, bladder, ovaries, uterus, or spleen (which may be the result, for example, of respiration or movement of the subject) or variations in blood flow (such as due to a pulse). Thus, in some embodiments, the implantable device is useful for tracking movement of an organ or a pulse rate. Movement of the implantable device can be tracked, for example, by monitoring changes in the non-responsive backscatter waves. In some embodiments, movement of the implantable device is determined my comparing the relative location of the implantable device at a first time point to the relative location of the implantable device at a second time point. For example, as described above, the location of an implantable device can be determined using a plurality of transducers on the interrogated (which may be on a single array or on two or more arrays). A first location of the implantable device can be determined at a first time point, and a second location of the implantable device can be determined at a second time point, and a movement vector can be determined based on the first location at the first time point and the second location at the second time point.

In some embodiments, the implantable device includes a clock, which can be calibrated or synced by the interrogator. For example, the interrogator can transmit a signal using the transmitted ultrasonic waves that sets or syncs the clock. The signal can be simultaneously transmitted to a plurality of implantable devices, thereby syncing the clocks of the plurality of implantable devices. In some embodiments, backscatter from the implantable devices encodes a timestamp based on the clock, which can indicate the time (or relative time) of an event (such as encountered radiation). This can be useful, for example to compare two or more implantable devices and the relative time of encountering radiation.

Implantable Device

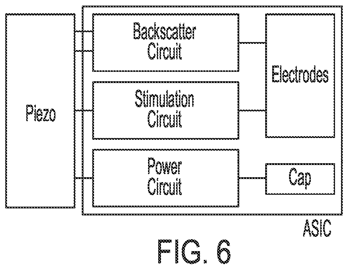

FIG. 6 illustrates one embodiment of a miniaturized ultrasonic transducer (identified as the "piezo") connected to an ASIC. The ASIC includes a power circuit and an optional modulation circuit (or "backscatter circuit"). The power circuit includes an energy storage capacitor ("cap"). Additionally, the implantable device includes a stimulation circuit (e.g., a digital circuit), which can operate the power circuit and the electrodes, which are implanted in or positioned against the tissue to be stimulated.

The implantable devices are miniaturized, which allows for comfortable and long-term implantation while limiting tissue inflammation that is often associated with implantable devices. The body forms the core of the miniaturized implantable device (e.g., the ultrasonic transducer and the integrated circuit), and the electrodes branch from the body and engage the tissue to deliver an electrical pulse to stimulate the tissue. In some embodiments, the longest dimension of the implantable device or the body of the implantable device is about 5 mm or less, about 4 mm or less, about 3 mm or less, about 2 mm or less, about 1 mm or less, about 0.5 mm or less, or about 0.3 mm or less in length. In some embodiments, the longest dimension of the implantable device or body of the implantable device is about 0.2 mm or longer, about 0.5 mm or longer, about 1 mm or longer, about 2 mm or longer, or about 3 mm or longer in the longest dimension of the device. In some embodiments, the longest dimension of the implantable device or the body of the implantable device is about 0.2 mm to about 5 mm in length, about 0.3 mm to about 4 mm in length, about 0.5 mm to about 3 mm in length, about 1 mm to about 3 mm in length, or about 2 mm in length.

In some embodiments, one or more of the electrodes are on the body of the device, for example a pad on the body of the device. In some embodiments, one or more of the electrodes extend from the body of the implantable device at any desired length, and can be implanted at any depth within the tissue. In some embodiments, an electrode is about 0.1 mm in length or longer, such as about 0.2 mm or longer, about 0.5 mm or longer, about 1 mm in length or longer, about 5 mm in length or longer, or about 10 mm in length or longer. In some embodiments, the electrodes are about 15 mm or less in length, such as about 10 mm or less, about 5 mm or less, about 1 mm or less, or about 0.5 mm or less in length. In some embodiments, the first electrode is disposed on the body of the implantable device and the second electrode extends from the body of the implantable device.

In some embodiments, the implantable device has a volume of about 5 mm or less (such as about 4 mm 3 or less, 3 mm 3 or less, 2 mm 3 or less, or 1 mm 3 or less). In certain embodiments, the implantable device has a volume of about 0.5 mm 3 to about 5 mm 3 , about 1 mm 3 to about 5 mm 3 , about 2 mm 3 to about 5 mm 3 , about 3 mm 3 to about 5 mm 3 , or about 4 mm 3 to about 5 mm . The small size of the implantable device allows for implantation of the device using a biopsy needle.

In some embodiments, the implantable device is implanted in a subject. The subject can be for example, an animal, such as a mammal. In some embodiments, the subject is a human, dog, cat, horse, cow, pig, sheep, goat, chicken, monkey, rat, or mouse. In some embodiments, the subject is a plant. Implantable devices implanted in plants can be useful, for example, for monitoring conditions of agricultural plants.

In some embodiments, the implantable device or a portion of the implantable device (such as the miniaturized ultrasonic transducer and the integrated circuit) is encapsulated by a biocompatible material (such as a biocompatible polymer), for example a copolymer of N-vinyl-2-pyrrolidinone (NVP) and n-butylmethacrylate (BMA), polydimethylsiloxane (PDMS), parylene, polyimide, silicon nitride, silicon dioxide, silicon carbide, alumina, niobium, or hydroxyapatite. The silicon carbide can be amorphous silicon carbide or crystalline silicon carbide. The biocompatible material is preferably impermeable to water to avoid damage or interference to electronic circuitry within the device. In some embodiments, the implantable device or portion of the implantable device is encapsulated by a ceramic (for example, alumina or titania) or a metal (for example, steel or titanium). In some embodiments, the electrodes or a portion of the electrodes are not encapsulated by the biocompatible material.

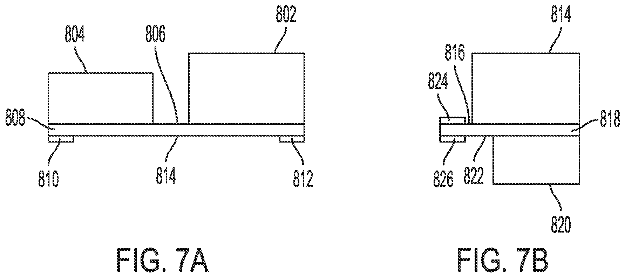

In some embodiments, the miniaturized ultrasonic transducer and the ASIC are disposed on a printed circuit board (PCB). The electrodes can optionally be disposed on the PCB, or can otherwise be connected to the integrated circuit. FIGS. 7A and 7B illustrate exemplary configurations of the implantable device including a PCB. FIG. 7A shows the piezoelectric transducer 802 and an ASIC 804 disposed on a first side 806 of the PCB 808. A first electrode 810 and a second electrode 812 are disposed on a second side 814 of the PCB 808. FIG. 7B sows the piezoelectric transducer 814 on a first side 816 of the PCB 818, and the ASIC 820 on the second side 822 of the PCB 818. A first electrode 824 initiates on the first side 816 of the PCB 818, and a second electrode 826 initiates on the second side 822 of the PCB 818. The first electrode 824 and the second electrode 826 can extend from the PCB 818 to be configured to be in electrical connection with each other through the tissue.

The miniaturized ultrasonic transducer of the implantable device can be a bulk piezoelectric transducer. Bulk piezoelectric transducers can be any natural or synthetic material, such as a crystal, ceramic, or polymer. Exemplary bulk piezoelectric transducer materials include barium titanate (BaTi03), lead zirconate titanate (PZT), zinc oxide (ZO), aluminum nitride (A1N), quartz, berlinite (AIPO4), topaz, langasite (La3Ga5SiOi4), gallium orthophosphate (GaP04), lithium niobate (LiNb03), lithium tantalite (LiTa03), potassium niobate (KNb03), sodium tungstate (Na2W03), bismuth ferrite (BiFe03), polyvinylidene (di)fluoride (PVDF), and lead magnesium niobate-lead titanate (PMN-PT).

In some embodiments, the miniaturized bulk piezoelectric transducer is approximately cubic (i.e., an aspect ratio of about 1 : 1 : 1 (length:width:height). In some embodiments, the piezoelectric transducer is plate-like, with an aspect ratio of about 5:5: 1 or greater in either the length or width aspect, such as about 7:5: 1 or greater, or about 10: 10: 1 or greater. In some embodiments, the miniaturized bulk piezoelectric transducer is long and narrow, with an aspect ratio of about 3: 1 : 1 or greater, and where the longest dimension is aligned to the direction of propagation of the carrier ultrasound wave. In some embodiments, one dimension of the bulk piezoelectric transducer is equal to one half of the wavelength ($\lambda$) corresponding to the drive frequency or resonant frequency of the transducer. At the resonant frequency, the ultrasound wave impinging on either the face of the transducer will undergo a 180° phase shift to reach the opposite phase, causing the largest displacement between the two faces. In some embodiments, the height of the piezoelectric transducer is about 10 $\mu m$ to about 1000 $\mu\pi$ (such as about 40 $\mu\pi$ to about 400 $\mu\pi$, about 100 $\mu\pi$ to about 250 $\mu\pi$, about 250 $\mu\pi$ to about 500 $\mu\pi$, or about 500 $\mu\pi$ to about 1000 $\mu\pi$). In some embodiments, the height of the piezoelectric transducer is about 5 mm or less (such as about 4 mm or less, about 3 mm or less, about 2 mm or less, about 1 mm or less, about 500 $\mu\pi$ or less, about 400 $\mu\pi$ or less, 250 $\mu\pi$ or less, about 100 $\mu\pi$ or less, or about 40 $\mu\pi$ or less). In some embodiments, the height of the piezoelectric transducer is about 20 $\mu\pi$ or more (such as about 40 $\mu\pi$ or more, about 100 $\mu\pi$ or more, about 250 $\mu\pi$ or more, about 400 $\mu\pi$ or more, about 500 $\mu\pi$ or more, about 1 mm or more, about 2 mm or more, about 3 mm or more, or about 4 mm or more) in length.

In some embodiments, the ultrasonic transducer has a length of about 5 mm or less such as about 4 mm or less, about 3 mm or less, about 2 mm or less, about 1 mm or less, about 500 $\mu\pi$ or less, about 400 $\mu\pi$ or less, 250 $\mu\pi$ or less, about 100 $\mu\pi$ or less, or about 40 $\mu\pi$ or less) in the longest dimension. In some embodiments, the ultrasonic transducer has a length of about 20 $\mu\pi$ or more (such as about 40 $\mu\pi$ or more, about 100 $\mu\pi$ or more, about 250 $\mu\pi$ or more, about 400 $\mu\pi$ or more, about 500 $\mu\pi$ or more, about 1 mm or more, about 2 mm or more, about 3 mm or more, or about 4 mm or more) in the longest dimension.

The miniaturized ultrasonic transducer is connected two electrodes; the first electrode is attached to a first face of the transducer and the second electrode is attached to a second face of the transducer, wherein the first face and the second face are opposite sides of the transducer along one dimension. In some embodiments, the electrodes comprise silver, gold, platinum, platinum-black, poly(3,4-ethylenedioxythiophene (PEDOT), a conductive polymer (such as conductive PDMS or polyimide), or nickel. In some embodiments, the transducer is operated in shear-mode where the axis between the metallized faces (i.e., electrodes) of the transducer is orthogonal to the motion of the transducer.

Manufacture of an Implantable Device

The implantable devices can be manufactured by attaching a miniaturized ultrasonic transducer (such as a bulk piezoelectric transducer) to a first electrode on a first face of the piezoelectric transducer, and a second electrode to a second face of the piezoelectric transducer, wherein the first face and the second face are on opposite sides of the piezoelectric transducer. The first electrode and the second electrode can be attached to an application-specific integrated circuit (ASIC), which may be disposed on a printed circuit board (PCB). Attachment of the components to the PCB can include wirebonding, soldering, flip-chip bonding, or gold bump bonding. The ASIC can include one or more sensors.

Certain piezoelectric materials can be commercially obtained, such as metalized PZT sheets of varying thickness (for example, PSI-5A4E, Piezo Systems, Woburn, MA, or PZT 841, APC Internationals, Mackeyville, PA). In some embodiments, a piezoelectric material sheet is diced into a desired size, and the diced piezoelectric material is attached to the electrodes. In some embodiments, the electrodes are attached to the piezoelectric material sheet, and the piezoelectric material sheet is diced to the desired size with the electrodes attached to the piezoelectric material. The piezoelectric material can be diced using a dicing saw with a ceramic blade to cut sheets of the piezoelectric material into individualized piezoelectric transducer. In some embodiments, a laser cutter is used to dice or singulate the piezoelectric material. In some embodiments, patterned etching is used to dice or singulate the piezoelectric material.

Electrodes can be attached to the top and bottom of the faces of the piezoelectric transducers, with the distance between the electrodes being defined as the height of the piezoelectric transducer. Exemplary electrodes can comprise one or more of silver, gold, platinum, platinum-black, poly(3,4-ethylenedioxythiophene (PEDOT), a conductive polymer (such as conductive PDMS or polyimide), or nickel. In some embodiments, the electrode is attached to the piezoelectric transducer by electroplating or vacuum depositing the electrode material onto the face of the piezoelectric transducer. In some embodiments, the electrodes are soldered onto the piezoelectric transducer using an appropriate solder and flux. In some embodiments, the electrodes are attached to the piezoelectric transducer using an epoxy (such as a silver epoxy) or low-temperature soldering (such as by use of a solder paste).

In an exemplary embodiment, solder paste is applied to a pad on a printed circuit board (PCB), either before or after the ASIC is attached to the PCB. The size of the pad on the circuit board can depend on the desired size of the piezoelectric transducer. Solely by way of example, if the desired size of piezoelectric transducer is about 100 $\mu\pi$ x 100 $\mu\pi$ x 100 $\mu\pi$, the pad can be about 100 $\mu\pi$ x 100 $\mu\pi$. The pad functions as the first electrode for the implantable device. A piezoelectric material (which may be larger than the pad) is placed on the pad, and is held to the pad by the applied solder paste, resulting in a piezoelectric-PCB assembly. The piezoelectric-PCB assembly is heated to cure the solder paste, thereby bonding the piezoelectric transducer to the PCB. If the piezoelectric material is larger than the pad, the piezoelectric material is cut to the desired size, for example using a wafer dicing saw or a laser cutter. Non- bonded portions of the piezoelectric material (for example, the portions of the piezoelectric material that did not overlay the pad) are removed. A second electrode is attached to the piezoelectric transducer and the PCB, for example by forming a wirebond between the top of the piezoelectric transducer and the PCB, which completes the circuit. The wirebond is made using a wire made from any conductive material, such as aluminum, copper, silver, or gold.

The integrated circuit and the miniaturized ultrasonic transducer can be attached on the same side of the PCB or on opposite sides of the PCB. In some embodiments, the PCB is a flexible PCB, the integrated circuit and the ultrasonic transducer are attached to the same side of the PCB, and the PCB is folded, resulting in an implantable device in which the integrated circuit and the ultrasonic transducer are on opposite sides of the PCB.

Optionally, the device or a portion of the device is encapsulated in or a portion of the device is encapsulated in a biocompatible material (such as a biocompatible polymer), for example a copolymer of N-vinyl-2-pyrrolidinone (NVP) and n-butylmethacrylate (BMA), polydimethylsiloxane (PDMS, e.g., Sylgard 184, Dow Corning, Midland, MI), parylene, polyimide, silicon nitride, silicon dioxide, alumina, niobium, hydroxy apatite, or silicon carbide. The silicon carbide can be amorphous silicon carbide or crystalline silicon carbide. In some embodiments, the biocompatible material (such as amorphous silicon carbide) is applied to the device by plasma enhanced chemical vapor deposition (PECVD) or sputtering. PECVD may use precursors such as S1H4 and CH4 to generate the silicon carbide. In some embodiments, the implantable device or portion of the implantable device is encased in a ceramic (for example, alumina or titania) or a metal (for example, steel or titanium) suitable for medical implantation.

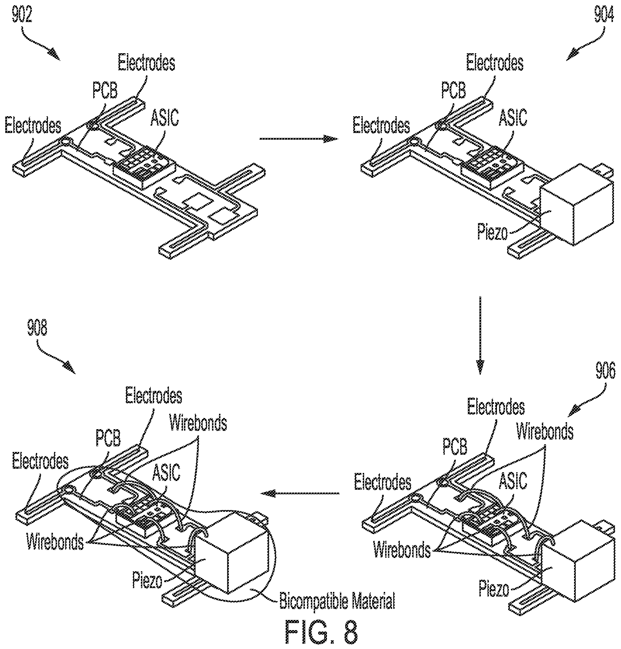

FIG. 8 illustrates an exemplary method of producing the implantable device described herein. At step 902, an application specific integrated circuit (ASIC) is attached to a PCB. The PCB can include two or more electrodes for detecting an electrophysiological signal in tissue. A solder (such as a silver epoxy) can be applied to the PCB (for example, at a first pad disposed on the PCB), and the ASIC can be placed on the solder. The solder can be cured, for example by heating the PCB with the ASIC. In some embodiments, the PCB with the ASIC is heated to about 50 °C to about 200 °C, such as about 80 °C to about 170 °C, or about 150 °C. In some embodiments, the PCB with the ASIC is heated for about 5 minutes to about 600 minutes, such as about 10 minutes to about 300 minutes, about 10 minutes to about 100 minutes, about 10 minutes to about 60 minutes, about 10 minutes to about 30 minutes, or about 15 minutes.

Optionally, the ASIC is coated with additional solder. At step 904, a piezoelectric transducer (the "piezo" in FIG. 8) is attached to the PCB. A solder (such as a silver epoxy) can be applied to the PCB (for example, at a second pad disposed on the PCB), and a piezoelectric material can be placed on the solder. The piezoelectric material can be a fully formed (i.e., "diced") piezoelectric transducer, or can be a piezoelectric material sheet that is cut to form the piezoelectric transducer once attached to the PCB. The solder can be cured, for example by heating the PCB with the piezoelectric material. In some embodiments, the PCB with the piezoelectric material is heated to about 50 °C to about 200 °C, such as about 80 °C to about 170 °C, or about 150 °C. In some embodiments, the PCB with the piezoelectric material is heated for about 5 minutes to about 600 minutes, such as about 10 minutes to about 300 minutes, about 10 minutes to about 100 minutes, about 10 minutes to about 60 minutes, about 10 minutes to about 30 minutes, or about 15 minutes. The piezoelectric material can be cut using a saw or laser cutter to the desired dimensions. In some embodiments, the piezoelectric material is a solgel (such as a PZT solgel) and the transducer material can be shaped with deep reactive ion etching (DRIE). Although FIG. 8 illustrates attachment of the ASIC to the PCB at step 902 prior to attachment of the piezoelectric material to the PCB at step 904, a person of skill in the art will appreciate that the ASIC and the piezoelectric material can be attached in any order. At step 906, the ASIC and the piezoelectric transducer are wirebonded to the PCB. Although the method illustrated in FIG. 8 shows the ASIC and the piezoelectric transducer to the PCB after the ASIC and the piezoelectric transducer are attached to the PCB, a person of skill in the art will appreciate that the ASIC can be wirebonded to the PCB after the ASIC is attached to the PCB, and can be wirebonded either before or after attachment of the piezoelectric transducer.

Similarly, the piezoelectric transducer may be wirebonded to the PCB either before or after attachment or wirebonding of the ASIC to the PCB. At step 908, at least a portion of the device is coated with a biocompatible material. Preferably, at least the piezoelectric transducer and the ASIC are coated with the biocompatible material. In some embodiments, the sensor is not or at least a portion of the sensor is not coated with the biocompatible material. For example, in some embodiments, the implantable device comprises a pair of electrodes which are not coated with the biocompatible material, which allows the electrodes to detect an electrophysiological signal. In some embodiments, the biocompatible material is cured, for example by exposure to UV light or by heating.

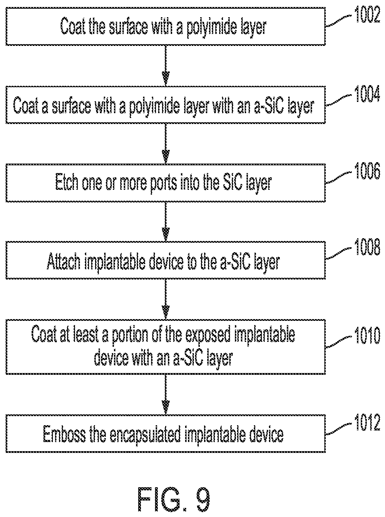

In some embodiments, the implantable device or a portion of the implantable device is encapsulated in an amorphous silicon carbide (a-SiC) film. FIG. 9 illustrates a method of manufacturing an implantable device encapsulated in an a-SiC film. At step 1002, a polyimide layer is applied to a smooth surface. At step 1004, an a-SiC layer is applied to the polyimide layer. This can be done, for example, using plasma enhanced chemical vapor deposition (PECVD), using SiH4 and CH4 as precursors. At step 1006, one or more ports are etched into the a-SiC layer. In some embodiments, ports are also etched into the polyimide layer. The ports provide access for portions of the implantable device that are not encapsulated by the a-SiC, such as portions of a sensor or an electrode that will contact the tissue after implant. In some embodiments, etching comprises reactive-ion etching. At step 1008, the implantable device is attached to the a-SiC layer. The implantable device may be pre-assembled before being attached to the a-SiC layer, or may be built on the a-SiC. In some embodiments, a printed circuit board (PCB), miniaturized ultrasonic transducer, and sensor are attached to the a-SiC layer. The miniaturized ultrasonic transducer and the sensor need not come in direct contact with the a-SiC layer, as they may be attached to the PCB. Attachment of miniaturized ultrasonic transducer or sensor to the PCB may occur before or after attachment of the PCB to the a-SiC layer. In some embodiments, attachment of miniaturized ultrasonic transducer or sensor to the PCB comprises wirebonding the miniaturized ultrasonic transducer or sensor to the PCB. In some embodiments, the sensor includes a portion that interfaces with the ports etched into the a-SiC layer. In some embodiments, an ASIC is attached to the PCB, which may occur before or after attachment of the PCB to the a-SiC layer. At step 1010, an exposed portion of the implantable device is coated with an a-SiC layer. In some embodiments, the exposed portion of the implantable device is coated with an a-SiC layer using PECVD. At step 1012, the encapsulated implantable device is embossed, thereby releasing the implantable device from the SiC layer.

Electrode Positioning to Reduce Energy Loss due to Anchor Loss

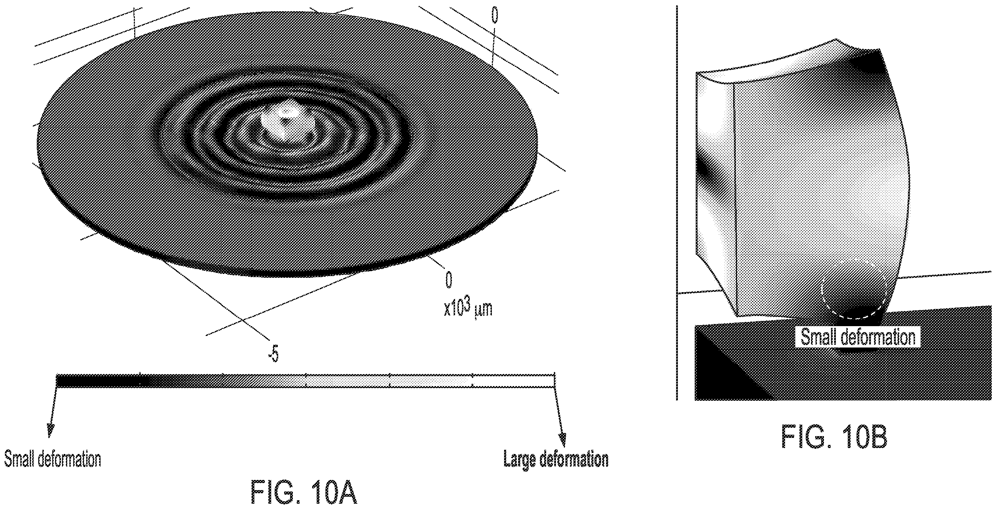

FIG. 10A is a heat map illustrating example deformation in a cubic piezoelectric crystal fully "anchored" to a substrate of an ultrasonic wireless implantable device (i.e., with an entire face of the cubic piezoelectric crystal anchored to the substrate). As shown in FIG. 10A, in this configuration, only limited deformation (i.e., limited vibration) of the cubic piezoelectric crystal is possible due to the cubic piezoelectric crystal being anchored to the substrate in this manner.

In contrast, FIG. 10B is a heat map illustrating example deformation in a cubic piezoelectric crystal anchored to the substrate of the ultrasonic wireless implantable device by an electrode positioned at the corners of one face of the cubic piezoelectric crystal. As shown in FIG. 10B, while limited deformation of the cubic piezoelectric crystal is possible at the corners of the face of the cubic piezoelectric crystal due to the anchoring electrodes, the rest of the cubic piezoelectric crystal is able to vibrate or oscillate more freely than when a substrate is attached directly to a full face of the cubic piezoelectric crystal. That is, while the corner electrodes restrict deformation/vibration at the corners of the cubic piezoelectric crystal, the corner electrodes allow for greater deformation in other portions of the cubic piezoelectric crystal compared to a substrate attached directly to a full face of the cubic piezoelectric crystal.

FIG. 11A, like FIG. 10B, is a heat map illustrating example deformation in a cubic piezoelectric crystal anchored to the substrate of the ultrasonic wireless implantable device by electrodes positioned at the corners of one face of the cubic piezoelectric crystal.

FIG. 11B illustrates an example of how electrodes could be positioned at the corners of one face of a cubic piezoelectric crystal to attach the face of the cubic piezoelectric crystal to a substrate. As shown in FIG. 11B, four electrodes, each with a length "L", are positioned at each of the four corners of the face of the cubic piezoelectric crystal. That is, the four electrodes shown in FIG. 11B may be attached to a substrate of an ultrasonic wireless implantable device to attach the piezoelectric crystal to the ultrasonic wireless implantable device without attaching the piezoelectric crystal directly to the substrate of the ultrasonic wireless implantable device.

FIG. 11C is an example graph illustrating a change in mechanical quality factor (Q) of the piezoelectric crystal as the length "L" of each corner electrode (as shown in FIG. 11B) is increased. As shown in FIG. 11C, the mechanical quality factor (Q) of the piezoelectric crystal generally decreases as the length of the corner electrodes increases.

FIG. 11D is an example graph illustrating a normalized amplitude of energy or voltage produced by a $1 \times 1 \times 1 \text{ mm}^3$ piezoelectric crystal attached to an ultrasonic wireless implantable device via corner electrodes (as shown in FIG. 11B) having various lengths "L" (and corresponding various mechanical factors (Q)) at a range of frequencies. The peaks for each corner electrode length "L" correspond to a maximum energy or voltage produced by the piezoelectric crystal for each corner electrode length "L" (e.g., at a resonant frequency of the piezoelectric crystal). As shown in FIG. 11D, the piezoelectric crystal attached to the corner electrodes having the shortest length "L" (100 $\mu$m) has the highest peak energy or voltage, while the piezoelectric crystal attached to the corner electrodes having the longest length "L" (350 μm) has the lowest peak energy or voltage.

FIG. 11E is an example graph illustrating a normalized amplitude of energy or voltage produced by a 0.75 x 0.75 x 0.75 mm³ piezoelectric crystal attached to an ultrasonic wireless implantable device via corner electrodes (as shown in FIG. 11B) having various lengths "L" (and corresponding various mechanical factors (Q)) at a range of frequencies. The peaks for each corner electrode length "L" correspond to a maximum energy or voltage produced by the piezoelectric crystal for each corner electrode length "L" (e.g., at a resonant frequency of the piezoelectric crystal). As shown in FIG. 11E, the piezoelectric crystal attached to the corner electrodes having the shortest length "L" (100 μm) has the highest peak energy or voltage, while the piezoelectric crystal attached to the corner electrodes having the longest length "L" (300 μm) has the lowest peak energy or voltage.

FIG. 12A is a heat map illustrating example deformation in a cubic piezoelectric crystal anchored to the substrate of the ultrasonic wireless implantable device by an electrode or a plurality of electrodes positioned at the edges of one face of the cubic piezoelectric crystal. As shown in FIG. 12A, while limited deformation of the cubic piezoelectric crystal is possible at the edges of the face of the cubic piezoelectric crystal due to the anchoring electrodes, the rest of the cubic piezoelectric crystal is able to vibrate or oscillate more freely than when a substrate is attached directly to a full face of the cubic piezoelectric crystal. That is, while the edge electrodes restrict deformation/vibration at the edges of the cubic piezoelectric crystal, the edge electrodes allow for greater deformation in other portions of the cubic piezoelectric crystal compared to a substrate attached directly to a full face of the cubic piezoelectric crystal.

FIG. 12B illustrates an example of how an electrode could be positioned at the edges of one face of a cubic piezoelectric crystal to attach the face of the cubic piezoelectric crystal to a substrate. As shown in FIG. 12B, a square or rectangular electrode, having a square or rectangular central opening with length "L", is attached to the face of the cubic piezoelectric crystal, such that the edges of the face of cubic piezoelectric crystal are attached to the electrode, while the center of the face of the cubic piezoelectric crystal is not touching the electrode (i.e., such that the edges of the face of the cubic piezoelectric crystal are "anchored," but the entire face of the cubic piezoelectric crystal is not fully anchored). That is, the edge electrode shown in FIG. 12B may be attached to a substrate of an ultrasonic wireless implantable device to attach the piezoelectric crystal to the ultrasonic wireless implantable device without attaching the piezoelectric crystal directly to the substrate of the ultrasonic wireless implantable device.

FIG. 12C is an example graph illustrating a change in mechanical quality factor (Q) of the piezoelectric crystal as the length "L" of the central opening in the electrode as shown in FIG. 12B is increased. As shown in FIG. 11C, the mechanical quality factor (Q) of the piezoelectric crystal generally increases as the length of the central opening in the electrode shown in FIG. 12B increases.

FIG. 12D is an example graph illustrating a normalized amplitude of energy or voltage produced by a 0.75 x 0.75 x 0.75 mm³ piezoelectric crystal attached to an ultrasonic wireless implantable device via an edge electrode (as shown in FIG. 12B) with a central opening having various lengths "L" (and corresponding various mechanical factors (Q)) at a range of frequencies. The peaks for each edge electrode central opening length "L" correspond to a maximum energy or voltage produced by the piezoelectric crystal for each edge electrode central opening length "L" (e.g., at a resonant frequency of the piezoelectric crystal). As shown in FIG. 12D, the piezoelectric crystal attached to the edge electrode with a central opening having the longest length "L" (500 μm) has the highest peak energy or voltage, while the piezoelectric crystal attached to the corner electrodes having the shortest length "L" (100 μm) has the lowest peak energy or voltage.

FIG. 13A is a heat map illustrating example deformation in a cubic piezoelectric crystal anchored to the substrate of the ultrasonic wireless implantable device by an electrode or a plurality of electrodes positioned at the center of one face of the cubic piezoelectric crystal. As shown in FIG. 13A, while limited deformation of the cubic piezoelectric crystal is possible at the center of the face of the cubic piezoelectric crystal due to the anchoring electrodes, the rest of the cubic piezoelectric crystal is able to vibrate or oscillate more freely than when a substrate is attached directly to a full face of the cubic piezoelectric crystal. That is, while the center electrodes restrict deformation/vibration at the center of the cubic piezoelectric crystal, the center electrodes allow for greater deformation in other portions of the cubic piezoelectric crystal compared to a substrate attached directly to a full face of the cubic piezoelectric crystal.

FIG. 13B illustrates an example of how an electrode could be positioned at the center of one face of a cubic piezoelectric crystal to attach the face of the cubic piezoelectric crystal to a substrate. As shown in FIG. 13B, a square or rectangular electrode having a length "L" is attached to the face of the cubic piezoelectric crystal, such that the electrode is positioned at approximately the center of the face of the cubic piezoelectric crystal. That is, the center electrode shown in FIG. 13B may be attached to a substrate of an ultrasonic wireless implantable device to attach the piezoelectric crystal to the ultrasonic wireless implantable device without attaching the piezoelectric crystal directly to the substrate of the ultrasonic wireless implantable device.

FIG. 13C is an example graph illustrating a change in mechanical quality factor (Q) of the piezoelectric crystal as the length "L" of the center electrode (as shown in FIG. 13B) is increased. As shown in FIG. 13C, the mechanical quality factor (Q) of the piezoelectric crystal generally decreases as the length of the center electrode.

FIG. 13D is an example graph illustrating a normalized amplitude of energy or voltage produced by a 1 x 1 x 1 mm³ piezoelectric crystal attached to an ultrasonic wireless implantable device via various center electrodes (as shown in FIG. 13B) having various lengths "L" (and corresponding various mechanical factors (Q)) at a range of frequencies. The peaks for each center electrode length "L" correspond to a maximum energy or voltage produced by the piezoelectric crystal for each center electrode length "L" (e.g., at a resonant frequency of the piezoelectric crystal). As shown in FIG. 13D, the piezoelectric crystal attached to the center electrode having the shortest length "L" (100 $\mu$m) has the highest peak energy or voltage, while the piezoelectric crystal attached to the corner electrodes having the longest length "L" (700 $\mu$m) has the lowest peak energy or voltage.

FIG. 14A illustrates an example of how electrodes could be positioned at the corners of one face of a cubic piezoelectric crystal to attach the face of the cubic piezoelectric crystal to a substrate (e.g., as shown in FIG. 11B), with a comparison of the lengths of the electrodes to the length of a face of the cubic piezoelectric crystal. As shown in FIG. 14A, four electrodes, each with a length "L", are positioned at each of the four corners of the face of the cubic piezoelectric crystal, and each face of the cubic piezoelectric crystal has a length "$L_p$."

FIG. 14B illustrates an example of how an electrode could be positioned at the edges of one face of a cubic piezoelectric crystal to attach the face of the cubic piezoelectric crystal to a substrate (e.g., as shown in FIG. 12B), with a comparison of the length of a central opening in the electrode to the length of a face of the cubic piezoelectric crystal. As shown in FIG. 14B, a square or rectangular electrode, having a central opening with length "L" is attached to a face of the cubic piezoelectric crystal, and each face of the cubic piezoelectric crystal has a length of "$L_p$."

FIG. 14C illustrates an example of how an electrode could be positioned at the center of one face of a cubic piezoelectric crystal to attach the face of the cubic piezoelectric crystal to a substrate (e.g., as shown in FIG. 13B), with a comparison of the length of the center electrode to the length of a face of the cubic piezoelectric crystal. As shown in FIG. 14C, a square or rectangular electrode having a length "L" is attached to the face of the cubic piezoelectric crystal, such that the electrode is positioned at approximately the center of the face of the cubic piezoelectric crystal, and each face of the cubic piezoelectric crystal has a length of "$L_p$."

FIG. 14D is a chart of example mechanical quality factor (Q) for each type of electrode (corner, edge, and center) for various piezoelectric crystal thicknesses ("$L_p$"), and for various ratios of $L/L_p$. For instance, as shown in FIG. 14D, for a 1 x 1 x 1 mm$^3$ piezoelectric crystal attached to a substrate of an implantable ultrasonic wireless device via corner electrodes with a length of 0.1 mm (i.e., an $L/L_p$ ratio of 0.1 mm / 1.0 mm = 0.1), the mechanical quality factor (Q) will be 170. In contrast, as shown in FIG. 14D, for a 0.75 x 0.75 x 0.75 mm$^3$ piezoelectric crystal attached to a substrate of an implantable ultrasonic wireless device via an edge electrode with a central opening having a length of ~0.1 mm (i.e., an $L/L_p$ ratio of 0.1 mm / 0.75 mm = ~0.134), the mechanical quality factor (Q) will be 7.

Accordingly, based on the data shown in FIG. 14D, a higher mechanical quality factor (Q) appears to be associated with a lower $L/L_p$ ratio for electrodes where L corresponds to a size of electrode surface area in contact with a face of the cubic piezoelectric crystal (e.g., corner and center electrodes). Similarly, based on the data shown in FIG. 14D, a higher mechanical quality factor (Q) appears to be associated with a higher $L/L_p$ ratio for electrodes where L corresponds to the surface area of an opening in an electrode in contact with a face of the cubic piezoelectric crystal (e.g., the edge electrode). Thus, a smaller amount of electrode surface area in contact with the face of the cubic piezoelectric crystal appears to correspond to a higher mechanical quality factor (Q). Furthermore, as discussed with respect to FIGS. 11D, 11E, 12D, and 13D, a higher mechanical quality factor (Q) appears to correspond to a higher maximum amplitude of energy and/or voltage produced by the vibration or oscillation energy of the piezoelectric crystal. Thus, compared to a full face of a cubic piezoelectric crystal being directly attached to a substrate of a wireless ultrasonic implantable device, any amount of electrode surface area in contact with a face of the piezoelectric crystal that is smaller than the surface area of a full face of the cubic piezoelectric crystal will generally yield a higher mechanical quality factor (Q) for the piezoelectric crystal, and thus, advantageously, a higher maximum amplitude of energy and/or voltage produced by the vibration or oscillation energy of the piezoelectric crystal.

Design of Efficient Ultrasonic Power and Data Link for Miniaturized Implants

FIGS. 15A and 15B show a packaged 1 × 1 × 1 mm$^3$ lead zirconate titanate (PZT) piezoelectric crystal (APC841). The package was built on a 400 $\mu$m-thick FR4 PCB with copper electrodes coated by gold. To assemble the package, the piezo with silver electrodes was anchored to the PCB electrodes with conductive epoxy. The 3D-printed housing was then attached to the PCB using UV-curable epoxy and sealed by the same epoxy. Finally, the piezo top face was covered with ~20 $\mu$m-thick PDMS, insulating it from the environment.

The resonator Q is defined as the ratio of energy stored to energy dissipated per oscillation period. To achieve high *Q* in resonators, energy dissipation (loss) should be minimized. Energy loss in piezo resonators generally results from fluid damping, anchor loss, and intrinsic loss (loss within the crystal itself). Among these loss mechanisms, fluid damping, causing energy loss from the piezo to the fluid, can dominate for piezos operated in aqueous environments. Here, the piezos were tested in water, which has an acoustic impedance similar to soft tissue (~1.5 MRayls). To minimize the effect of fluid damping on the piezo Q, the crystal sidewalls were isolated from the liquid environment using the housing shown in FIGS. 15A and 15B.

Minimizing fluid damping allowed for the identification of the effect of the other dominant source of energy loss, which is anchor loss, on the piezo Q. Anchor loss is the result of mechanical wave propagation from the anchor into the substrate. In existing ultrasonic implants, the piezocrystal is directly attached to the package substrate through an anchor (electrode) that completely covers the piezo bottom surface, making the piezo strongly coupled to the substrate and hence resulting in high anchor loss. In contrast, small anchors rather than a single large anchor are used here. The anchors were attached to the piezo regions that exhibited the minimum deformation during operation. An FEM simulation of the principal mode when operating a piezo with a bulk excitation normal to the top and bottom of a fixed-free piezocrystal shows that the largest deformation occurs at the center of the piezo bottom and top surfaces (FIG. 15C). A perfectly matched layer (PML) was used as an absorbing layer to simulate anchor loss numerically (FIG. 15C). Note that the PML properties should be appropriately chosen to achieve high accuracy in the simulation results.

The normalized admittance curves measured in air (FIG. 16A) show two important results: i) the principal bulk mode of the piezo with a $1\times1$ mm$^2$ single electrode disappears due to anchor loss (the other modes suppress its vibration); and ii) reducing the corner electrode size decreases anchor loss and hence improves the piezo resonance Q ($Q_r$), resulting in higher admittance at resonance. FIG. 16B depicts the measured and theoretical $Q_r$ values for the piezos in air with different size corner electrodes, showing a similar trend. The difference in the measured and theoretical results can be mainly attributed to fabrication imperfections in the electrodes, leading to size and location mismatch between the same electrodes (FIG. 15A), and misalignment in the placement of the piezo on the electrodes. The theoretical $Q_r^{-1}$ equals $Q_i^{-1} + Q_a^{-1}$; where $Q_i$ and $Q_a$ are the quality factors determined by the piezo intrinsic loss and anchor loss, respectively. To capture the intrinsic loss, the admittance curve of the piezo suspended using two wires without any anchor was measured in air, showed a $Q_i$ of 145. The $Q_a$ values were determined by the anchor loss simulations. The theoretical values in FIG. 16B show that the piezo $Q_r$ is dominated by the intrinsic loss for electrode sizes $\leq 200$ $\mu$m and decreases with an increasing electrode size since the anchor loss starts to dominate over the intrinsic loss for electrodes > 200 $\mu$m.

TABLE I. Operative and material parameters of the tested piezocrystals in water.

| Parameter | Electrode dimensions | |
|---|---|---|
| | 200 $\mu$m | 400 $\mu$m |
| Resonance frequency, $f_r$ (MHz) | 1.352 | 1.424 |
| Resonance quality factor, $Q_r$ | 29.2 | 10.2 |
| Effective mass, $m_{eq}$ (kg) | $2.66\times10^{-6}$ | $2.075\times10^{-6}$ |
| Stiffness, $k_{eq}$ (N/m) | $1.92\times10^{8}$ | $1.68\times10^{8}$ |
| System coupling, $\kappa^2$ | 0.355 | 0.239 |
| Piezo coefficient, $d_{33}$ (pm·V$^{-1}$) | 300 | |
| Relative permittivity, $\varepsilon_{33}^{T}/\varepsilon_0$ | 1375 | |
| Piezo capacitance, $C_p$ (pF) | ~12.2 | |

Fig. 16C shows the admittance curves measured in water, revealing that the piezo with a smaller anchor still demonstrates a higher $Q_r$ in the presence of fluid damping. The operative parameters of the piezos with corner electrodes in water, detailed in Table I, are obtained through the FEM simulations and the analytical model in equation 4 below. Note that the operative parameters of the piezo with a single electrode cannot be obtained since its longitudinal bulk mode is suppressed by the other modes, making the resonance invisible. Interestingly, we found that reducing the anchor size also improves system electromechanical coupling ($\kappa^2$). This is because the longitudinal bulk mode of the piezo with a smaller anchor is less coupled to the lateral motion from the higher-order modes as it has a higher $Q_r$.

Here, the piezocrystal operates in the 33-mode as the direction of mechanical strain induced by acoustic pressure waves is identical to the electrical polarization direction (perpendicular to the surface electrodes). The piezo response to the excitation force F(t) in the longitudinal z-direction can be modeled by the following governing equations:

$$\ddot{z}(t) + \frac{\omega_r}{Q_r}\dot{z}(t) + \omega_r^2\, z(t) - \frac{\theta}{m_{eq}}\, v_p(t) = \frac{1}{m_{eq}}F(t) \tag{1}$$

$$C_p \dot{v}_p(t) + \frac{1}{Z_{cir}} v_p(t) + \theta \dot{z}(t) = 0 \qquad (2)$$

where z(t), $m_{eq}$, $\omega_r$, and $Q_r$ are the piezo's longitudinal displacement at time $t$, equivalent effective mass, resonance frequency, and quality factor at resonance. The dot represents the time derivative, $v_p(t)$ is the voltage across the piezo, $\theta$ is the electromechanical coupling term, $C_p$ is the piezo internal capacitance, and $Z_{cir}$ is the electrical load impedance. Equations 1 and 2 describe the mechanical and electrical behaviors of the piezo, respectively; these two domains are electromechanically coupled through the coupling term $\theta$ (FIG. 19A). Here, $Z_{cir}$ is a parallel combination of the load capacitance ($C_{cir}$) and resistance ($R_{cir}$), and F(t) (=$p$(t)$\times A$) is produced by the incident acoustic pressure $p$(t) applied to the piezo's free-end surface (A: surface area). The piezo is positioned beyond the focal depth of an external transducer in measurements; therefore, the p(t) amplitude is assumed to be uniform across the piezo surface in the derivations.

By substituting $z(t)=ze^{j\omega t}$, $v_p(t)=v_p e^{j\omega t}$ and $F(t)=Fe^{j\omega t}$ into equations 1 and 2, solving these equations simultaneously at steady state, and rearranging the terms, the electrical power delivered to $R_{cir}$ can be obtained as

$$P_{out} = \frac{|v_p|^2}{R_{cir}} = \frac{\omega_r}{k_{eq}} \frac{F^2 \alpha \kappa^2 \tilde{\omega}^2}{\left[(1-\tilde{\omega}^2) - \frac{\tilde{\omega}^2(\alpha+\gamma)}{Q_r}\right]^2 + \left[\tilde{\omega}(\alpha+\gamma)(1-\tilde{\omega}^2) + \tilde{\omega}\alpha\kappa^2 + \frac{\tilde{\omega}}{Q_r}\right]^2} \qquad (3)$$

where $k_{eq}$ is the equivalent stiffness, determining the piezo $\omega_r = \sqrt{k_{eq}/m_{eq}}$, and dimensionless terms are defined: $\kappa^2=\theta l (C_p k_{eq})$ is the system coupling term, $\alpha=\omega_r R_{cir} C_p$ and $y=\omega_r R_{cir} C_{cir}$ are the time constants, and $\tilde{\omega}=\omega/\omega_r$ is the normalized excitation frequency. The power harvesting efficiency of the piezo is defined as: $\eta=P_{out}/P_{in}$, where $P_{in}$ is the total acoustic power at the piezo surface that equals $p^2 l(\rho_0 c_0) \times A$ - $\rho_0$ and $c_0$ are the density and sound velocity of the medium.

To provide a better understanding of piezo performance, we also analyze the impedance matching efficiency between the piezo and the electrical load, $\eta_{match}= P_{out}/P_{av,el}$, where $P_{av,el}$ is the available electrical power in the system. The Thevenin equivalent circuit model of the piezo (FIG. 19B) can be used to derive an expression for $\eta_{match}$ that is $P_{out}/P_{av,el} = |Z_L|^2/(R_{cir} \times Re\{Z_L + Z_S\})$, where $Z_L = Z_{cir}\|C_p$ and $Z_S$ is the piezo motional impedance. $Z_S$ can be derived from equations 1 and 2 as follows for the piezo driven by an excitation voltage $v(t)= Ve^{j\omega t}$ in the absence of F(t) by replacing the terms $C_p \dot{v}_p(t)+Z_{cir}^{-1}v_p(t)$ by the actuation current input $-i(t) = -I_e^{j\omega t}$:

$$Z_S = V/I = \frac{k_{eq}}{\omega_r} \frac{\left[(1-\tilde{\omega}^2)+ j\frac{\tilde{\omega}}{Q_r}\right]}{j\tilde{\omega}\theta^2} \qquad (4)$$

$\eta_{match}$ can be derived using equation 4 as

$$\eta_{match} = \frac{\alpha\kappa^2}{\left[\alpha\kappa^2 + \frac{1}{Q_r} + \frac{\tilde{\omega}^2(\alpha+\gamma)^2}{Q_r}\right]} \qquad (5)$$

The piezocrystals were characterized in a water tank setup (Fig. 20), where an external transducer generated pressure waves and the transducer distance to the piezo was set to 3 cm, ensuring far-field operation. To evaluate the effect of piezo parameters, resulting from the use of different anchor sizes, on the harvesting efficiency $\eta$, the piezos packaged on 200$\times$200 $\mu$m and 400$\times$400 $\mu$m corner electrodes were operated at their resonance frequencies ($\omega_r$) with different load resistances ($R_{cir}$). FIG. 17A depicts the measured $\eta$ versus $R_{cir}$, showing perfect agreement with the $\eta$ predicted from the analytical models above. The optimal $R_{cir,opt} = 1/\omega_r C_p(\kappa^4 Q_r^2 + 1 + C_{cir}/C_p)$, obtained by differentiating equation 3 with respect to $R_{cir}$ and equating the resulting expression to zero, gives the maximum $\eta$ ($\eta_{max}$) at $\omega_r$. Note that $C_{cir}$ is ~6 pF, including the unavoidable parasitic capacitance from wiring and probe capacitance, in the measurements, which is smaller than $C_p$ of ~12.2 pF. For the strongly coupled, high-$Q_r$ piezo with 200 $\mu$m anchors, the $R_{cir,opt}$ simplifies to $1/(\kappa^2 Q_r \omega_r C_p)$ due to $\kappa^4 Q_r^2 \gg 1 + C_{cir}/C_p$, yielding $\eta_{max}\approx P_{in}^{-1}\times(\omega_r/k_{eq} \times Q_r/4)$ which is independent of the system coupling $\kappa^2$. For the weakly coupled, low-$Q_r$ piezo with 400 $\mu$m anchors, $\eta_{max}$ also depends on $\kappa^2$; a smaller $\kappa^2$ results in $\eta_{max}<P_{in}^{-1} \times(\omega_r/k_{eq} \times Q_r/4)$ at $R_{cir,opt}$[17]. Therefore, the piezo with smaller anchors at $\omega_r$ and $R_{cir,opt}$ provides substantially higher $\eta_{max}$ since reducing the anchor size improves both $Q_r$ and $\kappa^2$.

FIGS. 21A and 21B illustrate the power harvesting efficiencies obtained at various $R_{cir}$ versus the normalized excitation frequency ($\tilde{\omega}$) for the piezos with different size anchors. As $R_{cir}$ is increased, the piezo resonance frequency shifts from $\omega_r$ to anti-resonance $\omega_a$ due to electromechanical coupling. For the piezo with 200 $\mu$m anchors, two peaks with similar $\eta$ values were determined, one at $\omega_r$ and the other at $\omega_a$, but for the piezo with 400 $\mu$m anchors, only one-peak was observed between $\omega_r$ and $\omega_a$. This is because the piezo with larger anchors is weakly coupled as it has substantially lower $\kappa^2$ and $Q_r$. Overall, the piezo with smaller anchors provides higher $\eta$ in a range between $\omega_r$ and $\omega_a$ since it is strongly coupled and has a higher $Q_r$. Furthermore, the $\eta$ obtained by the piezo with a $1\times1$ mm$^2$ single electrode (Fig. S4) is more than $5.4\times$ lower than the $\eta$ achieved at $\omega_r$ by the piezo with 200 $\mu$m anchors in the tested frequency range.

FIG. 17B shows the predicted matching efficiencies ($\eta_{match}$) of the piezos at $\omega_r$. From equation 5,

$$R_{cir,opt} \approx 1/\widetilde{\omega}\omega_r\left(C_p + C_{cir}\right) \text{ and } \eta_{match,max} = \kappa^2\Big/\left(\kappa^2 + \frac{2\widetilde{\omega}}{Q}\frac{(C_p+C_{cir})}{C_p}\right),$$

revealing that the parameters that determine the $R_{cir,opt}$ for achieving $\eta_{max}$ and $\eta_{match,max}$ appear different. Therefore, the $\eta_{max}$ and $\eta_{match,max}$ may occur at different $R_{cir,opt}$ for the piezo operated at $\omega_r$ (FIGS. 3A and 3B).The maximum power transfer occurs when the load and source impedances ($Z_S$ and $Z_L$ in Fig. S1(b)) are matched, resulting in $\eta_{match}$=0.5. For the strongly coupled, high-$Q_r$ piezo, there are two intersection points where $\eta_{match}$=0.5; the first point corresponds to the $R_{cir,opt}$ providing the $\eta_{max}$ at $\omega_r$, and the second point indicates the other $R_{cir,opt}$ providing the $\eta_{max}$ at $\omega_a$ (see FIG. 21A). This observation reveals the two peaks with similar $\eta_{max}$ at $\omega_r$ and $\omega_a$ (see FIG. 21A). For the weakly coupled, low-$Q_r$ piezo, the $\eta_{match}$ value is smaller than 0.5 at any $R_{cir}$ value, indicating that perfect impedance matching is not achievable and the maximum power transfer occurs at the $R_{cir,opt}$ providing $\eta_{match,max}$ (closest to $\eta_{match}$=0.5). Here, the $R_{cir,opt}$, providing $\eta_{match,max}$ is different than the $R_{cir,opt}$ providing the $\eta_{max}$ at $\omega_r$. This explains the observation of a single $\eta$ peak between $\omega_r$ and $\omega_a$ at the $R_{cir,opt}$, providing $\eta_{match,max}$ and hence $\eta_{max}$ at the same time (Fig. 21B).

The backscatter amplitude modulation is achieved by changing the load impedance ($Z_{cir}$) as the acoustic reflection coefficient $\Gamma$ at the piezo boundary is a function of $Z_{cir}$, that is $P_r = \Gamma(Z_{cir})^2 \times P_{in}$, where $P_r$ is the reflected acoustic power from the piezo surface. The maximum modulation depth, defined as the ratio of the amplitude difference between the modulated and unmodulated signals to the unmodulated signal, is obtained at resonance ($\omega_r$). Here, the effect of system parameters on $\Gamma$ at $\omega_r$ were investigated for the piezos with different anchor sizes and different $R_{cir}$ values. The $\Gamma$ can be predicted at $\omega_r$ using its Thevenin equivalent circuit model (FIG. 18 and 19B) as follows:

$$|\Gamma| \approx \left|\frac{Z_L}{Z_L+Z_s}\right| \approx \frac{\alpha\kappa^2}{\sqrt{\left(\frac{\alpha+\gamma}{Q_r}\right)^2 + \left(\alpha\kappa^2 + \frac{1}{Q_r}\right)^2}} \tag{6}$$

where $Z_L=Z_{cir}\|C_p$. Equation 6 reveals that $\Gamma$ depends on not only $R_{cir}$ (through $\alpha$ and $\gamma$), but also quality factor $Q_r$ and system coupling term $\kappa^2$.

FIG. 18 depicts the measured and predicted $\Gamma$ of the piezos with 200 $\mu$m and 400 $\mu$m anchors, showing that the experimental data agree well with the predicted data using equation 6. Note that the measured $\Gamma$ was obtained by inserting the collected data points into an equation: $\Gamma \approx \frac{V_b(R_{cir}) - V_b(R_{cir} \approx 0)}{V_b(R_{cir} \approx \infty) - V_b(R_{cir} \approx 0)}$, where $V_b$ is the backscatter signal detected by the external transducer. As $R_{cir}$ increases, $\Gamma$ approaches 1 for the strongly coupled, high-$Q_r$ piezo, but it is not able to reach 1 for the weakly coupled, low-$Q_r$ piezo. The individual effect of $O_r$ and $\kappa^2$ on $\Gamma$ at $\omega_r$ is demonstrated in FIGS. 23A and 23B, showing that any decrease in either $Q_r$ or $\kappa^2$ moves the $\Gamma$ curve to the right and reduces the maximum achievable $\Gamma$ value. Therefore, a decrease in either $Q_r$ or $\kappa^2$ also reduces the modulation depth (MD) and hence acoustic link signal-to-noise ratio, degrading system uplink performance. For example, modulating $R_{cir}$ between 500 $\Omega$ and 100 k$\Omega$ results in a MD of ~10% for the weakly coupled, low-$Q_r$ piezo, significantly lower than the MD (~230%) achieved by the strongly coupled high-$Q_r$ piezo (FIGS. 24A and 24B).

Accordingly, an attachment method for piezocrystals used in mm- and sub-mm scale medical implants is provided herein. The method relies on placing small anchors at piezocrystal regions where the excited mode displacement is minimal; this approach reduces anchor loss, maximizing the piezocrystal's *Q-factor.* The results of this study also indicate that the use of small anchors maximizes system coupling. The study further shows that the strongly coupled high-Q piezo with small corner electrodes achieves ~10$\times$ higher power harvesting efficiency $\eta$ (~48%) at resonance than that of a piezo with a single electrode (covering all of the bottom side of the piezocrystal), operated at the same excitation frequency (FIGS. 21A, 21B, and 22). Additionally, the study shows that a strongly coupled high-Q piezo achieves a high backscatter modulation depth at resonance, demonstrating a robust uplink. Overall, these results show that a piezocrystal packaged as described herein, operated with an optimal $R_{load}$, provides an advantage for wireless systems with ultra-miniature ultrasonic implants.

Furthermore, when implemented, any of the methods and techniques described herein or portions thereof may be performed by executing software stored in one or more non-transitory, tangible, computer readable storage media or memories such as magnetic disks, laser disks, optical discs, semiconductor memories, biological memories, other memory devices, or other storage media, in a RAM or ROM of a computer or processor, etc.

Of course, the applications and benefits of the systems, methods and techniques described herein are not limited to only the above examples. Many other applications and benefits are possible by using the systems, methods and techniques described herein.

**Claims**

1. An implantable device, comprising:

   a substrate;
   an integrated circuit attached to the substrate; and
   an ultrasonic transducer configured to receive ultrasonic waves that power the integrated circuit, wherein the ultrasonic transducer is attached to the substrate, **characterised in that** the ultrasonic transducer is attached to the substrate via one or more electrodes, and wherein the total electrode surface area in contact with the ultrasonic transducer is smaller than the surface area of a face of the ultrasonic transducer to which the one or more electrodes are attached.

2. The implantable device of claim 1, wherein:

   (a) the one or more electrodes are each positioned at a respective corner of the face of the ultrasonic transducer to which the one or more electrodes are attached;
   (b) the one or more electrodes are each positioned at an edge of the face of the ultrasonic transducer to which the one or more electrodes are attached;
   (c) the one or more electrodes comprise a single electrode positioned at a center of the face of the ultrasonic transducer to which the single electrode is attached; or
   (d) the one or more electrodes include an electrode having an opening between the face of the ultrasonic transducer and the substrate.

3. The implantable device of claim 1, wherein:

   (a) the ultrasonic transducer has a cubic shape; or
   (b) the ultrasonic transducer has a rectangular prism shape.

4. The implantable device of claim 1, wherein the face of the ultrasonic transducer to which the one or more electrodes are attached has a rectangular shape.

5. The implantable device of claim 1, wherein:

   (a) a ratio of a length of each of the one or more electrodes to a length of the face of the ultrasonic transducer to which the one or more electrodes are attached is less than 0.1; or
   (b) a ratio of a length of each of the one or more electrodes to a length of the face of the ultrasonic transducer to which the one or more electrodes are attached is less than 0.2.

6. The implantable device of claim 1, wherein the ultrasonic transducer is further configured to emit an ultrasonic backscatter.

7. The implantable device of claim 1, wherein the ultrasonic transducer is configured to receive the ultrasonic waves from an interrogator comprising one or more additional ultrasonic transducers.

8. The implantable device of claim 1, wherein the ultrasonic transducer is a bulk piezoelectric transducer.

9. The implantable device of claim 1, wherein the implantable device is implantable in a subject, wherein:

   (a) the subject is a human; or
   (b) the subject is an animal or a plant.

10. The implantable device of claim 1, wherein the implantable device is about 5 mm or less in length in the longest dimension.

11. The implantable device of claim 1, wherein the implantable device has a volume of about 5 mm or smaller.

12. The implantable device of claim 1, wherein the implanted device is at least partially encapsulated by a biocompatible material.

13. A system comprising one or more implantable devices according to claim 1, and an interrogator comprising one or more ultrasonic transducers configured to transmit ultrasonic waves to the one or more implantable devices or receive ultrasonic backscatter from the one or more implantable devices.

14. The system according to claim 13, wherein the interrogator comprises one or more ultrasonic transducer arrays, wherein each transducer array comprises two or more ultrasonic transducers.

15. The system according to claim 13, wherein:

(a) the system comprises a plurality of implantable devices;
(b) the interrogator is configured to beam steer transmitted ultrasonic waves to alternatively focus the transmitted ultrasonic waves on a first portion of the plurality of implantable devices or focus the transmitted ultrasonic waves on a second portion of the plurality of implantable devices;
(c) the interrogator is configured to simultaneously receive ultrasonic backscatter from at least two implantable devices;
(d) the interrogator is configured to transit ultrasonic waves to the plurality of implantable devices or receive ultrasonic backscatter from the plurality of implantable devices using time division multiplexing, spatial multiplexing, or frequency multiplexing; or
(e) the interrogator is configured to be wearable by a subject.

**Patentansprüche**

1. Implantierbare Vorrichtung, umfassend:

ein Substrat;
eine integrierte Schaltung, die an dem Substrat angebracht ist; und
einen Ultraschallwandler, der ausgelegt ist, um Ultraschallwellen, die die integrierte Schaltung mit Leistung versorgen, zu empfangen, wobei der Ultraschallwandler an dem Substrat angebracht ist, **dadurch gekennzeichnet, dass** der Ultraschallwandler über eine oder mehrere Elektroden an dem Substrat angebracht ist, und wobei die Gesamtelektrodenoberfläche, die mit dem Ultraschallwandler in Kontakt steht, kleiner ist als ein Oberflächenbereich einer Fläche des Ultraschallwandlers, an der die eine oder die mehreren Elektroden angebracht sind.

2. Implantierbare Vorrichtung nach Anspruch 1, wobei:

(a) die eine oder die mehreren Elektroden jeweils in einer jeweiligen Ecke der Fläche des Ultraschallwandlers, an dem die eine oder die mehreren Elektroden angebracht sind, positioniert sind;
(b) die eine oder die mehreren Elektroden jeweils an einem Rand der Fläche des Ultraschallwandlers positioniert sind, an der die eine oder die mehreren Elektroden angebracht sind;
(c) die eine oder die mehreren Elektroden eine einzelne Elektrode umfassen, die an einem Mittelpunkt der Fläche des Ultraschallwandlers positioniert ist, an der die einzelne Elektrode angebracht ist; oder
(d) die eine oder die mehreren Elektroden eine Öffnung zwischen der Fläche des Ultraschallwandlers und des Substrats aufweisen.

3. Implantierbare Vorrichtung nach Anspruch 1, wobei:

(a) der Ultraschallwandler eine würfelförmige Form aufweist; oder
(b) der Ultraschallwandler eine rechteckige Prismaform aufweist.

4. Implantierbare Vorrichtung nach Anspruch 1, wobei die Fläche des Ultraschallwandlers, an die die eine oder die mehreren Elektroden angebracht sind, eine rechteckige Form aufweist.

5. Implantierbare Vorrichtung nach Anspruch 1, wobei:

(a) ein Verhältnis einer Länge jeder der einen oder der mehreren Elektroden zu einer Länge der Fläche des Ultraschallwandlers, an der die eine oder die mehreren Elektroden angebracht sind, weniger als 0,1 beträgt; oder
(b) ein Verhältnis der Länge jeder der einen oder der mehreren Elektroden zu einer Länge der Fläche des Ultraschallwandlers, an der die eine oder die mehreren Elektroden angebracht sind, weniger als 0,2 beträgt.

6. Implantierbare Vorrichtung nach Anspruch 1, wobei der Ultraschallwandler weiters ausgelegt ist, um eine Ultraschallrückstreuung auszustrahlen.

7. Implantierbare Vorrichtung nach Anspruch 1, wobei der Ultraschallwandler ausgelegt ist, um die Ultraschallwellen von einem Abfrageelement zu empfangen, das einen oder mehrere zusätzliche Ultraschallwandler umfasst.

8. Implantierbare Vorrichtung nach Anspruch 1, wobei der Ultraschallwandler ein piezoelektrischer Volumswandler ist.

9. Implantierbare Vorrichtung nach Anspruch 1, wobei die implantierbare Vorrichtung in ein Individuum implantierbar ist, wobei:

   (a) das Individuum ein Mensch ist; oder
   (b) das Individuum ein Tier oder eine Pflanze ist.

10. Implantierbare Vorrichtung nach Anspruch 1, wobei die längste Abmessung der implantierbaren Vorrichtung eine Länge von etwa 5 mm oder weniger aufweist.

11. Implantierbare Vorrichtung nach Anspruch 1, wobei die implantierbare Vorrichtung ein Volumen von etwa 5 mm oder kleiner aufweist.

12. Implantierbare Vorrichtung nach Anspruch 1, wobei die implantierbare Vorrichtung zumindest teilweise durch ein biokompatibles Material eingekapselt ist.

13. System, umfassend eine oder mehrere implantierbare Vorrichtungen nach Anspruch 1, und ein Abfrageelement, umfassend einen oder mehrere Ultraschallwandler, die ausgelegt sind, um Ultraschallwellen an die eine oder die mehreren implantierbaren Vorrichtungen zu übertragen oder Ultraschallrückstreuung von der einen oder den mehreren implantierbaren Vorrichtungen zu empfangen.

14. System nach Anspruch 13, wobei das Abfrageelement eine oder mehrere Ultraschallwandleranordnungen umfasst, wobei jede Wandleranordnung zwei oder mehr Ultraschallwandler umfasst.

15. System nach Anspruch 13, wobei:

   (a) das System eine Vielzahl von implantierbaren Vorrichtungen umfasst;
   (b) das Abfrageelement ausgelegt ist, um übertragene Ultraschallwellen mittels Strahllenkung zu lenken, um die übertragenen Ultraschallwellen alternativ auf einen ersten Abschnitt aus der Vielzahl von implantierbaren Vorrichtungen zu fokussieren oder die übertragenen Ultraschallwellen auf einen zweiten Abschnitt aus der Vielzahl von implantierbaren Vorrichtungen zu fokussieren;
   (c) das Abfrageelement ausgelegt ist, um gleichzeitig Ultraschallrückstreuung von zumindest zwei implantierbaren Vorrichtungen zu empfangen;
   (d) das Abfrageelement ausgelegt ist, um Ultraschallwellen an die Vielzahl von implantierbaren Vorrichtungen zu übertragen oder Ultraschallrückstreuung von der Vielzahl von implantierbaren Vorrichtungen unter Verwendung von Zeitteilungsmultiplexing, Raummultiplexing oder Frequenzmultiplexing zu empfangen; oder
   (e) das Abfrageelement ausgelegt ist, um durch ein Individuum tragbar zu sein.

**Revendications**

1. Dispositif implantable, comprenant :

   un substrat ;
   un circuit intégré fixé au substrat ; et
   un transducteur ultrasonore configuré pour recevoir des ondes ultrasonores qui alimentent le circuit intégré, dans lequel le transducteur ultrasonore est fixé au substrat, **caractérisé en ce que**

le transducteur ultrasonore est fixé au substrat via une ou plusieurs électrodes, et dans lequel la surface d'électrode totale en contact avec le transducteur ultrasonore est plus petite que la surface d'une face du transducteur ultrasonore à laquelle les une ou plusieurs électrodes sont fixées.

2. Dispositif implantable selon la revendication 1, dans lequel :

   (a) les une ou plusieurs électrodes sont positionnées chacune au niveau d'un coin respectif de la face du transducteur ultrasonore à laquelle les une ou plusieurs électrodes sont fixées ;
   (b) les une ou plusieurs électrodes sont positionnées chacune au niveau d'un bord de la face du transducteur ultrasonore à laquelle les une ou plusieurs électrodes sont fixées ;
   (c) les une ou plusieurs électrodes comprennent une électrode unique positionnée au centre de la face du transducteur ultrasonore à laquelle l'électrode unique est fixée ; ou
   (d) les une ou plusieurs électrodes incluent une électrode présentant une ouverture entre la face du transducteur ultrasonore et le substrat.

3. Dispositif implantable selon la revendication 1, dans lequel :

   (a) le transducteur ultrasonore présente une forme cubique ; ou
   (b) le transducteur ultrasonore présente une forme de prisme rectangulaire.

4. Dispositif implantable selon la revendication 1, dans lequel la face du transducteur ultrasonore à laquelle les une ou plusieurs électrodes sont fixées présente une forme rectangulaire.

5. Dispositif implantable selon la revendication 1, dans lequel :

   (a) un rapport entre une longueur de chacune des une ou plusieurs électrodes et une longueur de la face du transducteur ultrasonore à laquelle les une ou plusieurs électrodes sont fixées est inférieur à 0,1 ; ou
   (b) un rapport d'une longueur de chacune des une ou plusieurs électrodes à une longueur de la face du transducteur ultrasonore à laquelle les une ou plusieurs électrodes sont fixées est inférieur à 0,2.

6. Dispositif implantable selon la revendication 1, dans lequel le transducteur ultrasonore est en outre configuré pour émettre une rétrodiffusion ultrasonore.

7. Dispositif implantable selon la revendication 1, dans lequel le transducteur ultrasonore est configuré pour recevoir les ondes ultrasonores en provenance d'un interrogateur comprenant un ou plusieurs transducteurs ultrasonores supplémentaires.

8. Dispositif implantable selon la revendication 1, dans lequel le transducteur ultrasonore est un transducteur piézoélectrique en vrac.

9. Dispositif implantable selon la revendication 1, dans lequel le dispositif implantable est implantable chez un sujet, dans lequel :

   (a) le sujet est un être humain ; ou
   (b) le sujet est un animal ou une plante.

10. Dispositif implantable selon la revendication 1, dans lequel le dispositif implantable présente une longueur d'environ 5 mm ou moins dans la dimension la plus longue.

11. Dispositif implantable selon la revendication 1, dans lequel le dispositif implantable présente un volume d'environ 5 mm ou moins.

12. Dispositif implantable selon la revendication 1, dans lequel le dispositif implanté est au moins partiellement encapsulé par un matériau biocompatible.

13. Système comprenant un ou plusieurs dispositifs implantables selon la revendication 1, et un interrogateur comprenant un ou plusieurs transducteurs ultrasonores configurés pour transmettre des ondes ultrasonores aux un ou plusieurs dispositifs implantables, ou pour recevoir une rétrodiffusion ultrasonore à partir des un ou plusieurs dispositifs implantables.

14. Système selon la revendication 13, dans lequel l'interrogateur comprend un ou plusieurs réseaux de transducteurs ultrasonores, dans lequel chaque réseau de transducteurs comprend deux transducteurs ultrasonores ou plus.

15. Système selon la revendication 13, dans lequel :

(a) le système comprend une pluralité de dispositifs implantables ;
(b) l'interrogateur est configuré pour diriger par faisceau des ondes ultrasonores transmises afin de focaliser alternativement les ondes ultrasonores transmises sur une première partie de la pluralité de dispositifs implantables ou de focaliser les ondes ultrasonores transmises sur une seconde partie de la pluralité de dispositifs implantables ;
(c) l'interrogateur est configuré pour recevoir simultanément une rétrodiffusion ultrasonore à partir d'au moins deux dispositifs implantables ;
(d) l'interrogateur est configuré pour transmettre des ondes ultrasonores à la pluralité de dispositifs implantables ou pour recevoir une rétrodiffusion ultrasonore à partir de la pluralité de dispositifs implantables en utilisant un multiplexage par répartition dans le temps, un multiplexage spatial ou un multiplexage fréquentiel ; ou
(e) l'interrogateur est configuré pour pouvoir être porté par un sujet.

FIG. 1

FIG. 2A

EP 4 106 863 B1

User Interface

Storage Medium

Input/Output

Power Supply

Integrated
Circuit

FIG. 2B

FIG. 3A

FIG. 3C

FIG. 3E

FIG. 3B

FIG. 3D

32

FIG. 4

FIG. 5A

FIG. 5C

FIG. 5B

FIG. 5D

FIG. 5E

FIG. 6

FIG. 7A

FIG. 7B

FIG. 8

```
┌────────────────────────────────────────────┐
│      Coat the surface with a polyimide layer       │───1002
└────────────────────────────────────────────┘
                      │
                      ▼
┌────────────────────────────────────────────┐
│  Coat a surface with a polyimide layer with an a-SiC layer  │───1004
└────────────────────────────────────────────┘
                      │
                      ▼
┌────────────────────────────────────────────┐
│        Etch one or more ports into the SiC layer        │───1006
└────────────────────────────────────────────┘
                      │
                      ▼
┌────────────────────────────────────────────┐
│       Attach implantable device to the a-SiC layer       │───1008
└────────────────────────────────────────────┘
                      │
                      ▼
┌────────────────────────────────────────────┐
│  Coat at least a portion of the exposed implantable   │───1010
│             device with an a-SiC layer               │
└────────────────────────────────────────────┘
                      │
                      ▼
┌────────────────────────────────────────────┐
│      Emboss the encapsulated implantable device      │───1012
└────────────────────────────────────────────┘
```

FIG. 9

Small deformation ← → Large deformation

FIG. 10A

Small deformation

FIG. 10B

EP 4 106 863 B1

FIG. 11A

FIG. 11B

FIG. 11C

1x1x1 mm³ PZT crystal (APC841)
Q = 170 ⋯ L = 100μm
Q = 115 ⋯ L = 200μm
Q = 50 ⋯ L = 350μm

FIG. 11D

0.75x0.75x0.75 mm³ PZT crystal (APC841)
Q = 134 ⋯ L = 100μm
Q = 86 ⋯ L = 200μm
Q = 53 ⋯ L = 350μm

FIG. 11E

FIG. 12C

FIG. 12D

FIG. 12A

FIG. 12B

FIG. 13A

FIG. 13B

FIG. 13C

1x1x1 mm³ PZT crystal (APC841)

Q = 98    L = 100μm
Q = 76    L = 300μm
Q = 16    L = 700μm

FIG. 13D

EP 4 106 863 B1

FIG. 14A

FIG. 14B

FIG. 14C

| Electrode type | Piezo thickness (mm) | L/L_p | Quality factor (Q) |
|---|---|---|---|
| Corner | 1 | 0.1 | 170 |
| | 1 | 0.2 | 115 |
| | 1 | 0.35 | 50 |
| Center | 1 | 0.1 | 98 |
| | 1 | 0.3 | 76 |
| | 1 | 0.7 | 16 |
| Corner | 0.75 | 0.134 | 134 |
| | 0.75 | 0.267 | 86 |
| | 0.75 | 0.4 | 53 |
| Edge | 0.75 | 0.67 | 71 |
| | 0.75 | 0.4 | 55 |
| | 0.75 | 0.134 | 7 |

* Mechanical Q of the PZT (APC841) = 1400

FIG. 14D

EP 4 106 863 B1

Bottom electrodes

Piezo crystal top face

970 μm

370 μm

370 μm

FR4 PCB    Piezo housing

FIG. 15A

UV curable epoxy

Piezo housing

Air gap

PCB

PDMS

Bottom electrodes

FIG. 15B

PML

FIG. 15C

Max disp

Corner electrode

FR4 PCB

Min disp

FIG. 15D

EP 4 106 863 B1

FIG. 16A

FIG. 16B

FIG. 16C

FIG. 17A

FIG. 17B

FIG. 18

FIG. 19A

FIG. 19B

EP 4 106 863 B1

48

Olympus, V306-SU
F0 .80IN-PTF

FIG. 20

FIG. 21B

FIG. 21A

FIG. 22

FIG. 23B

FIG. 23A

FIG. 24B

FIG. 24A

**EP 4 106 863 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019204773 A
- US 20190150883
- US 20190150882
- US 20190150884
- US 10300310 B
- US 10300309 B
- US 20190150881
- US 10118054 B

**Non-patent literature cited in the description**

- **BERTRAND et al.** Beamforming Approaches for Untethered, Ultrasonic Neural Dust Motes for Cortical Recording: a Simulation Study. *IEEE EMBC*, August 2014